(19) Europäisches Patentamt
European Patent Office
Office européen des brevets

(11) Veröffentlichungsnummer: **0 264 551**
**B1**

(12) **EUROPÄISCHE PATENTSCHRIFT**

(45) Veröffentlichungstag der Patentschrift: **09.01.91**

(51) Int. Cl.[5]: **C 07 C 271/06,** C 08 F 20/36, A 61 K 6/08

(21) Anmeldenummer: **87110819.7**

(22) Anmeldetag: **14.07.87**

(54) **Urethangruppen enthaltende (Meth)-acrylsäurederivate.**

(30) Priorität: **25.07.86 DE 3625202**
**03.02.87 DE 3703130**

(43) Veröffentlichungstag der Anmeldung:
**27.04.88 Patentblatt 88/17**

(45) Bekanntmachung des Hinweises auf die Patenterteilung:
**09.01.91 Patentblatt 91/02**

(84) Benannte Vertragsstaaten:
**AT BE CH DE ES FR GB IT LI NL SE**

(56) Entgegenhaltungen:
**GB-A-1 328 232**

(73) Patentinhaber: **BAYER AG**
**D-5090 Leverkusen 1 Bayerwerk (DE)**

(72) Erfinder: **Reiners, Jürgen, Dr.**
**Carl-Rumpff-Strasse 57**
**D-5090 Leverkusen 1 (DE)**
Erfinder: **Podszun, Wolfgang, Dr.**
**Wolfskaul 4**
**D-5000 Koeln 80 (DE)**
Erfinder: **Winkel, Jens, Dr.**
**Hahnenweg 6**
**D-5000 Koeln 80 (DE)**
Erfinder: **Süling, Carlhans**
**Carl-Leverkus-Strasse 10**
**D-5068 Odenthal (DE)**
Erfinder: **Klein, Gerhard, Dr.**
**von-Flotow-Strasse 7**
**D-4019 Monheim (DE)**

**EP 0 264 551 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft neue Urethangruppen enthaltende (Meth)-acrylsäurederivate, ihre Herstellung und ihre Verwendung als monomere Komponenten für Dentalwerkstoffe.

Die Verwendung von polyfunktionellen (Meth)-acrylsäurederivaten als Komponenten für Zahnfüllungsmaterialien ist bekannt. So werden in der EP—A 0 017 936 Acrylsäureester und Meth-acrylsäureester von Pentaerylthrit beschrieben. Die dort beschriebenen Monomeren ergeben in Kombination mit anorganischen Füllstoffen Dentalwerkstoffe, die einen unerwünschten Polymerisationsschrumpf aufweisen, der zu einer Spaltbildung zwischen Zahn- und Füllungsmaterial führt.

In der US 45 54 336 werden Urethangruppen enthaltende (Meth)-acrylsäurederivate für Adhesive im Dentalbereich beschrieben, bei denen die Urethangruppen durch einen eine (Meth)-acrylatgruppe enthaltenden Rest substituiert sind. Diese Verbindungen zeigen als Komponenten in Dentalmassen unzureichende Eigenschaften, insbesondere eine für die Praxis zu geringe Festigkeit.

Es wurden neue Urethangruppen enthaltende (Meth)-acrylsäurederivate der Formel (I)

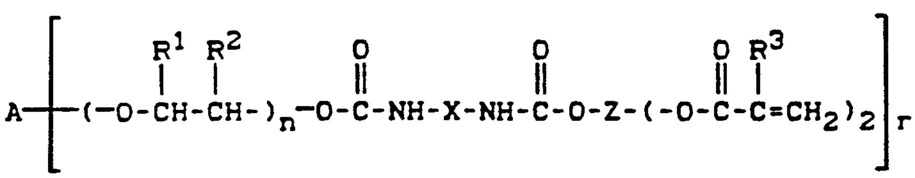 (I),

in der

A ein geradkettiger oder verzweigter, gegebenenfalls 1 bis 3 Sauerstoffbrücken enthaltender, aliphatischer Rest mit 2 bis 20 Kohlenstoffatomen, ein aromatischer Rest mit 6 bis 24 Kohlenstoffatomen, ein araliphatischer Rest mit 7 bis 26 Kohlenstoffatomen oder ein cycloaliphatischer Rest mit 6 bis 26 kohlenstoffatomen ist,

r für die Anzahl der von A ausgehenden Ketten steht und eine Zahl von 2 bis 6 bedeutet,

$R^1$ und $R^2$ gleich sind und Wasserstoff oder verschieden sind und Wasserstoff und Methyl bedeuten,

n für jede von A ausgehende Kette unabhängig eine Zahl von 0 bis 5 bedeutet,

X ein zweiwertiger, geradkettiger oder verzweigter aliphatischer Rest mit 2 bis 24 Kohlenstoffatomen, ein aromatischer Rest mit 6 bis 26 Kohlenstoffatomen, ein araliphatischer Rest mit 7 bis 26 Kohlenstoffatomen oder ein mono-cycloaliphatischer Rest mit 6 bis 26 Kohlenstoffatomen ist, wobei die aliphatischen, aromatischen, araliphatischen und/oder mono-cycloaliphatischen Reste 1 oder 2 Sauerstoffbrücken enthalten können, und wobei mehrere der aliphatischen, aromatischen, araliphatischen und/oder mono-cycloaliphatischen Reste über gegebenenfalls substituierte Methylengruppen verdunden sein können,

Z einen dreiwertigen geradkettigen oder verzweigten aliphatischen Kohlenwasserstoffrest mit 3 bis 15 Kohlenstoffatomen, der gegebenenfalls 1 bis 3 Sauerstoffbrücken enthalten kann und gegebenenfalls durch 1 bis 3 zusätzliche (Meth)-acrylatreste substituiert sein kann, bedeutet, und

$R^3$ für jede von A ausgehende Kette unabhängig Wasserstoff oder Methyl bedeutet, gefunden.

Dentalwerkstoffe, bei denen von den erfindungsgemäßen Urethangruppen enthaltenden (Meth)-acrylsäurederivaten ausgegangen wird, zeigen überraschenderweise einen wesentlich geringeren Polymerisationsschrumpf und größere Festigkeit, und sind daher für die Anwendung in der Praxis besonders geeignet.

Im Rahmen der vorliegenden Erfindung können die Substituenten folgende Bedeutung haben:

Ein aliphatischer Rest (A) kann ein geradkettiger oder verzweigter Kohlenwasserstoffrest mit 2 bis 20, bevorzugt 3 bis 12, Kohlenstoffatomen sein. Beispielsweise seien die folgenden aliphatischen Reste genannt:

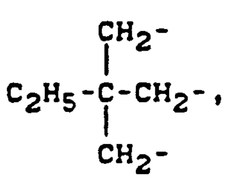 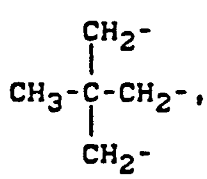 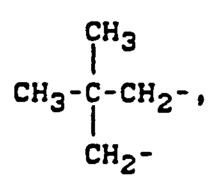 $-CH_2-CH_2-$,

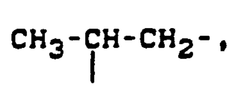 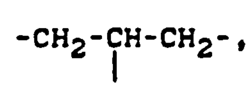 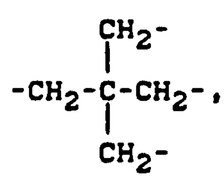

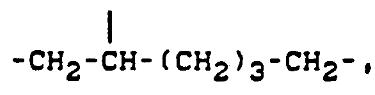, 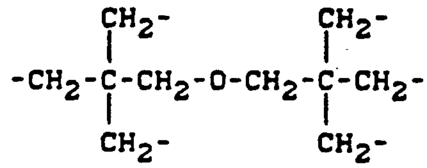

Ein aromatischer Rest (A) kann ein aromatischer Kohlenwasserstoffrest mit 6 bis 24, bevorzugt 6 bis 14, Kohlenstoffatomen sein. Beispielsweise seien die folgenden aromatischen Reste genannt:

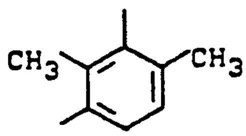, 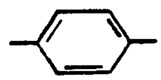, 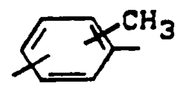,

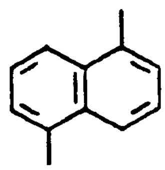, 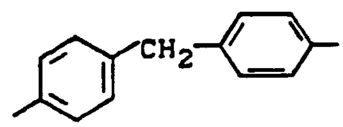

Ein araliphatischer Rest (A) kann ein Kohlenwasserstoffrest mit einem geradkettigen oder verzweigten aliphatischen und einem aromatischen Teil mit 7 bis 26 Kohlenstoffatomen sein, wobei der aromatische Teil bevorzugt 6 bis 12 und der aliphatische Teil bevorzugt 1 bis 14 Kohlenstoffatome enthält. Beispielsweise seien die folgenden araliphatischen Reste genannt:

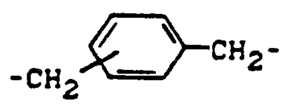, 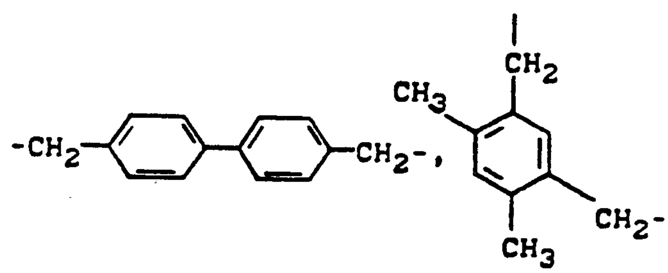,

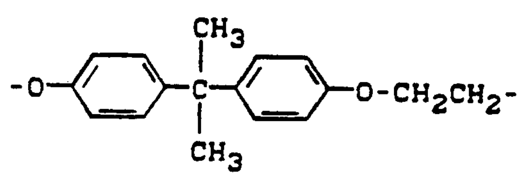,

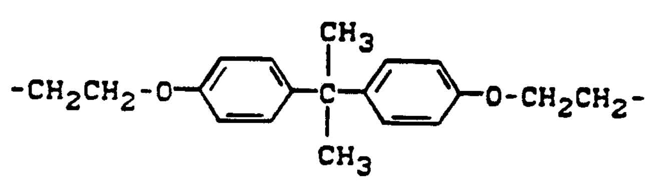,

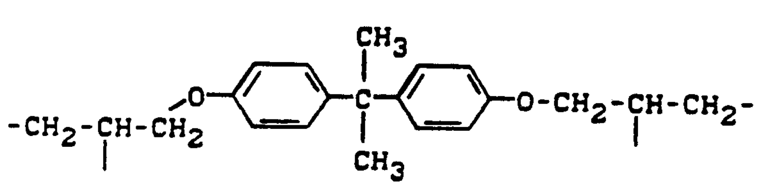

Ein cycloaliphatischen Rest (A) kann ein cyclischer Kohlenwasserstoffrest mit 6 bis 26 Kohlenstoffatomen, bevorzugt 6 bis 14 Kohlenstoffatomen, sein. Beispielsweise seien die folgenden cycloaliphatischen Reste genannt:

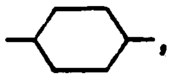, 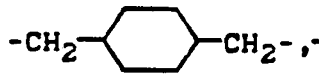, 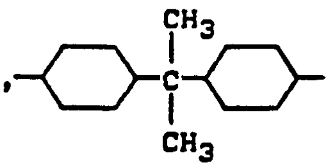,

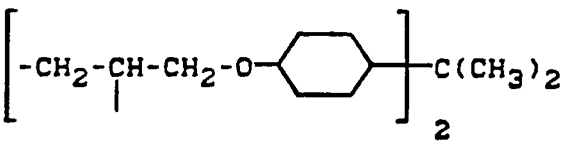, 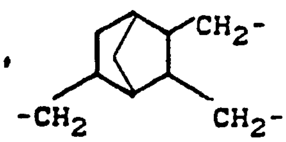,

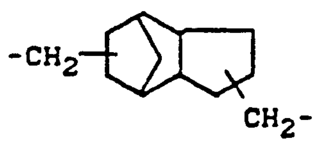, 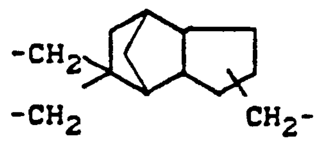,

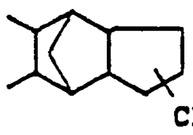, 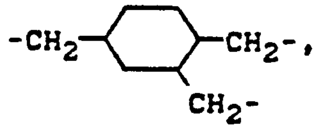, 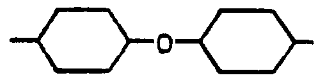

Die Reste A können, bevorzugt im aliphatischen oder cycloaliphatischen Teil, 1 oder 2, bevorzugt 1, Sauerstoffatome enthalten, so daß beispielsweise aliphatische bzw. cycloaliphatische Ether vorliegen.

Insbesondere bevorzugt seien die folgenden Reste A genannt: Ethylen, Propylen, 2,2-Bismethylen-butan-1-yl, 2,2-Bismethylen-propan-1-yl, 2,2-Bis-methylen-propan-1,3-diyl, 1,1'-Oxy-bis-[(2,2-methylen)-propan-1,3-diyl], Propan-1,2,3-triyl, 1,6-Hexamethylen, 1,4-Tetramethylen, 1,4-Phenylen, Xylylen, 1,4-Cyclohexylen, 1,4-Bismethylen-1,4-cyclohexan, 2,2-bis(1,4-phenylen)-propan, 3(4), 8(9)-Bismethylen-Tricyclo[5.2.1.$0^{2,6}$]decan und dessen Isomere, 4(5),9-Bismethylen-3,8-dimethyltricyclo [5.2.1.$0^{2,6}$]decan.

Insbesondere bevorzugt sind die Reste 2,2-Bismethylenbutan-1-yl, Propan-1,2,3-triyl, 2,2-Bismethylen-propan-1,3-diyl und 3(4),8(9)-Bismethylen-Tricyclo[5.2.1.0.$^{2,6}$]decan.

Ein zwierwertiger, geradkettiger oder verzweigter aliphatischer Rest (X) kann einen Kohlenwasserstoffrest mit 2 bis 24 Kohlenstoffatomen, bevorzugt 2 bis 16 Kohlenstoffatomen, bedeuten. Beispielsweise seien die folgenden zweiwertigen aliphatischen Reste genannt: Ethylen, Propylen, 1,4-Tetramethylen, 1,6-Hexamethylen oder 2,2,4-Trimethyl-1,6-hexamethylen und Isomere.

Ein zweiwertiger aromatischer Rest (X) kann ein aromatischer Kohlenwasserstoffrest mit 6 bis 26, bevorzugt 6 bis 18, Kohlenstoffatomen bedeuten. Beispielsweise seien die folgenden aromatischen Reste genannt:

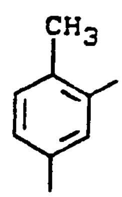, 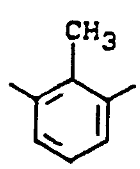, 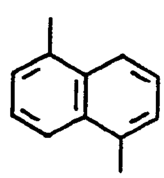, 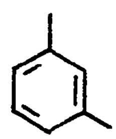,

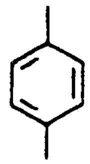 , 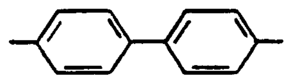 , 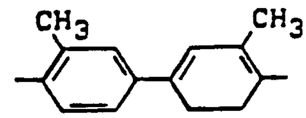 ,

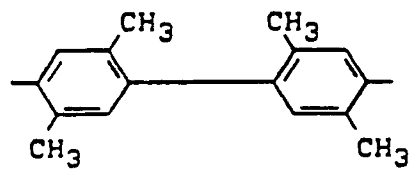 , 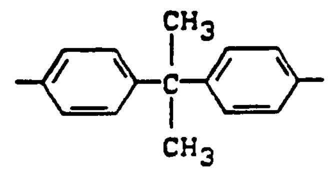 ,

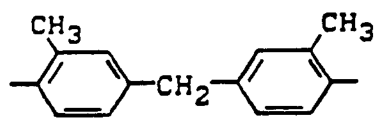 , 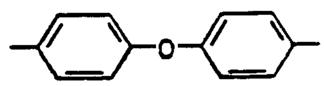 .

Ein zweiwertiger araliphatischer Rest (X) kann ein Kohlenwasserstoffrest mit einem geradkettigen oder verzweigten aliphatischen und einem aromatischen Teil mit 7 bis 20 Kohlenstoffatomen bedeuten, wobei der aromatische Teil bevorzugt 6 bis 12 und der aliphatische bevorzugt 1 bis 8 Kohlenstoffatome enthält. Beispielsweise seien die folgenden araliphatischen Reste genannt:

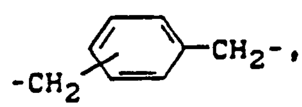 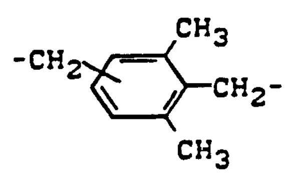

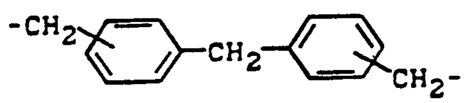 , 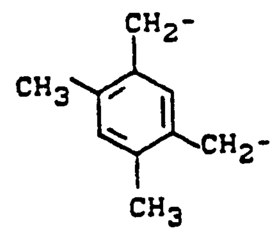

Ein zweiwertiger monocycloaliphatischer Rest (X) kann einen Kohlenwasserstoffrest mit 6 bis 26, bevorzugt 6 bis 14 Kohlenstoffatomen bedeuten. Beispielsweise seien genannt:

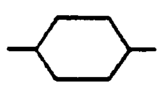 , 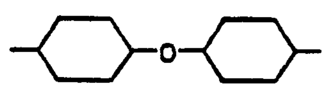 , 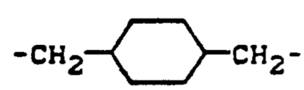 ,

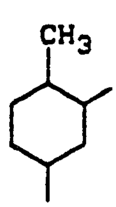 , 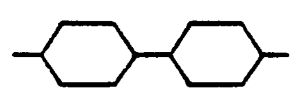 , 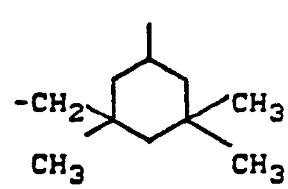

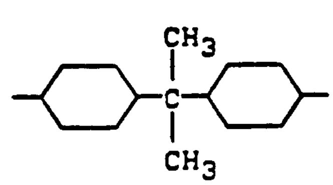 , 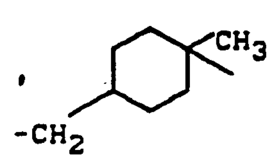 , 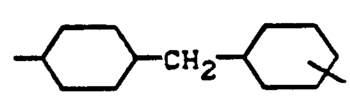

Es ist auch möglich, daß mehrere (vorzugsweise 1 bis 3) der genannten aromatische, araliphatischen und/oder mono cycloaliphatischen Rest über gegebenenfalls substituierte Methylengruppen verbunden sind.

Gegebenenfalls substituierte Methylengruppen sind die Gruppen

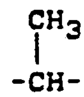 , 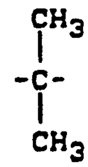 , 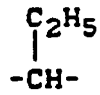 , 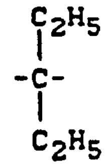 .

Ein dreiwertiger Kohlenwasserstoffrest (Z) kann ein geradkettiger oder verzweigter aliphatischer Kohlenwasserstoff mit 3 bis 15 Kohlenstoffatomen, bevorzugt 3 bis 10, Kohlenstoffatomen sein. Der Rest Z kann gegebenenfalls 1 bis 3 Sauerstoffbrücken, bevorzugt 1 bis 2 Sauerstoffbrücken enthalten. Es ist auch möglich, daß der Rest Z durch 1 bis 3, bevorzugt 1 bis 2 (Meth)acrylatreste substituiert ist. Beispielsweise seien die folgenden Reste genannt:

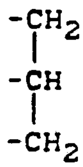 , 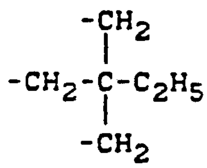 , 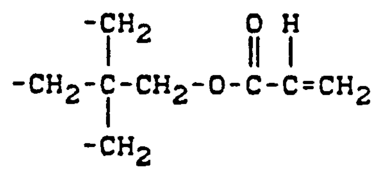 ,

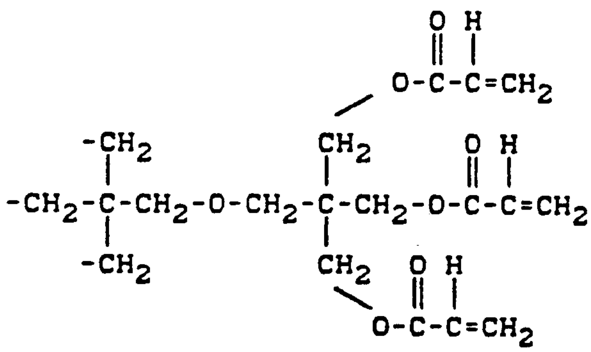 ,

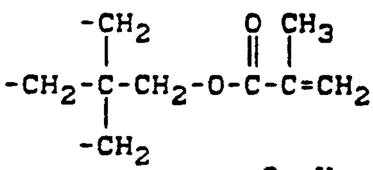 ,

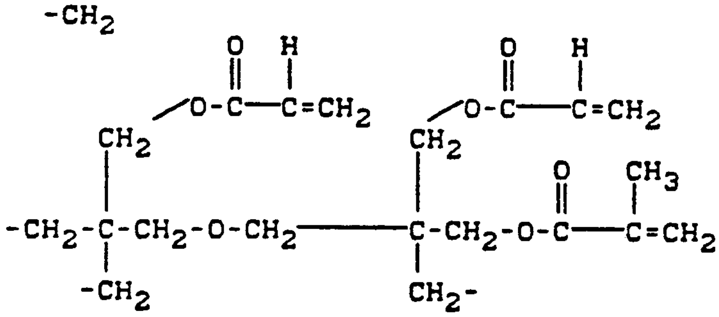 ,

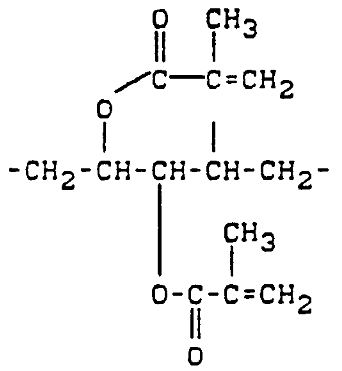
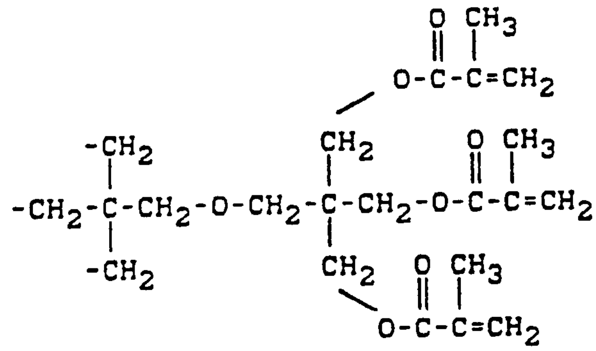
,

Bevorzugt werden Urethangruppen enthaltende (Meth)acrylsäurederivate der Formel (I), bei denen

A ein geradkettiger oder verzweigter, gegebenenfalls 1 bis 3 Sauerstoffbrücken enthaltender, aliphatischer Rest mit 3 bis 12 Kohlenstoffatomen, ein aromatischer Rest mit 6 bis 14 Kohlenstoffatomen, ein araliphatischer Rest mit 7 bis 26 Kohlenstoffatomen oder ein cycloaliphatischer Rest mit 6 bis 14 Kohlenstoffatomen ist,

r für die Anzahl der von A ausgehenden Ketten steht und eine Zahl von 2 bis 6 bedeutet,

$R^1$ und $R^2$ gleich sind und Wasserstoff oder verschieden sind und Wasserstoff und Methyl bedeuten,

n für jede von A ausgehende Kette unabhängig einer Zahl von 0 bis 5 bedeutet,

X ein zweiwertiger, geradkettiger oder verzweigter aliphatischer Rest mit 2 bis 12 Kohlenstoffatomen, oder ein monocycloaliphatischer Rest mit 6 bis 14 Kohlenstoffatomen, oder ein araliphatischer Rest mit 6 bis 18 Kohlenstoffatomen, wobei auch 1 bis 3 der aliphatischen, monocycloaliphatischen oder aromatischen Reste über gegebenenfalls substituierte Methylengruppen verbunden sein können, ist,

Z ein dreiwertiger geradkettiger oder verzweigter aliphatischer Kohlenwasserstoffrest mit 3 bis 10 Kohlenstoffatomen, der gegebenenfalls 1 oder 2 Sauerstoffbrücken enthalten kann und gegebenenfalls durch 1 oder 2 (Meth)-acrylatreste substituiert sein kann, ist, und

$R^3$ für jede von A ausgehende Kette unabhängig Wasserstoff oder Methyl bedeutet.

Insbesondere bevorzugt werden Urethangruppen enthaltende (Meth)-acrylsäurederivate der Formel (I), bei denen

A für den 2,2-Bismethylen-butan-1-yl-Rest, Propan-1,2,3-triyl-Rest, 2,2-Bismethylenpropan-1,3-diyl-Rest oder 3(4),8(9)-Bismethylen-tricyclo[5.2.1.$0^{2,6}$]decan-Rest steht,

r für die Anzahl der von A ausgehenden Ketten steht und die Zahl von 3 bis 4 bedeutet,

$R^1$ und $R^2$ gleich sind und Wasserstoff oder verschieden sind und Wasserstoff und Methyl bedeuten,

n für jede von A ausgehende Kette unabhängig einer Zahl von 0 bis 5 bedeutet,

X für einen der Reste

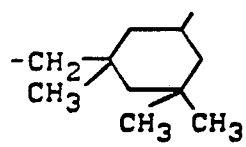

oder

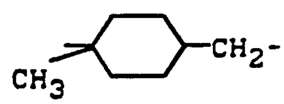

steht,

Z einen dreiwertigen geradkettigen oder verzweigten aliphatischen Kohlenwasserstoffrest mit 3 bis 10 Kohlenstoffatomen, der gegebenenfalls 1 Sauerstoffbrücke enthalten und gegebenenfalls durch 1 (Meth)acrylatrest substituiert sein kann, bedeutet, und

$R^3$ für jede von A ausgehende Kette unabhängig Wasserstoff oder Methyl bedeutet.
Beispielsweise seien die folgenden Urethangruppen enthaltenden (Meth)-acrylsäurederivate genannt:

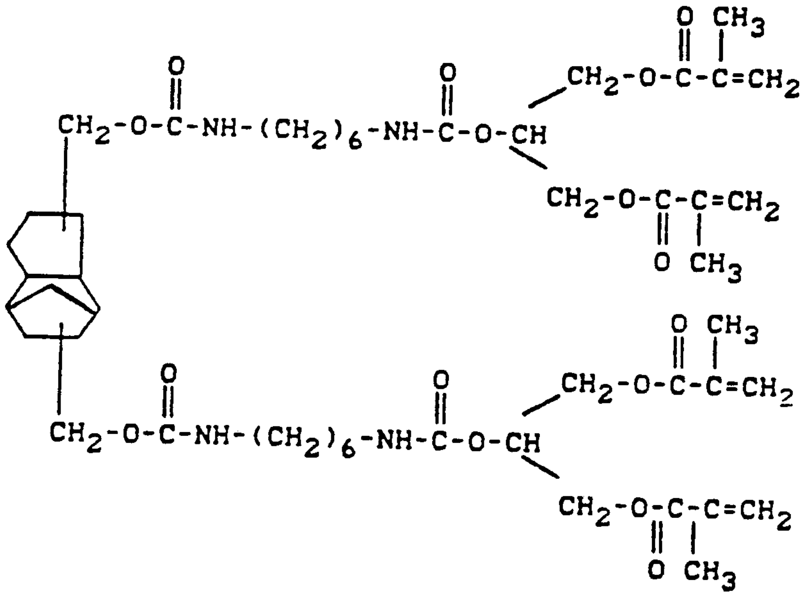

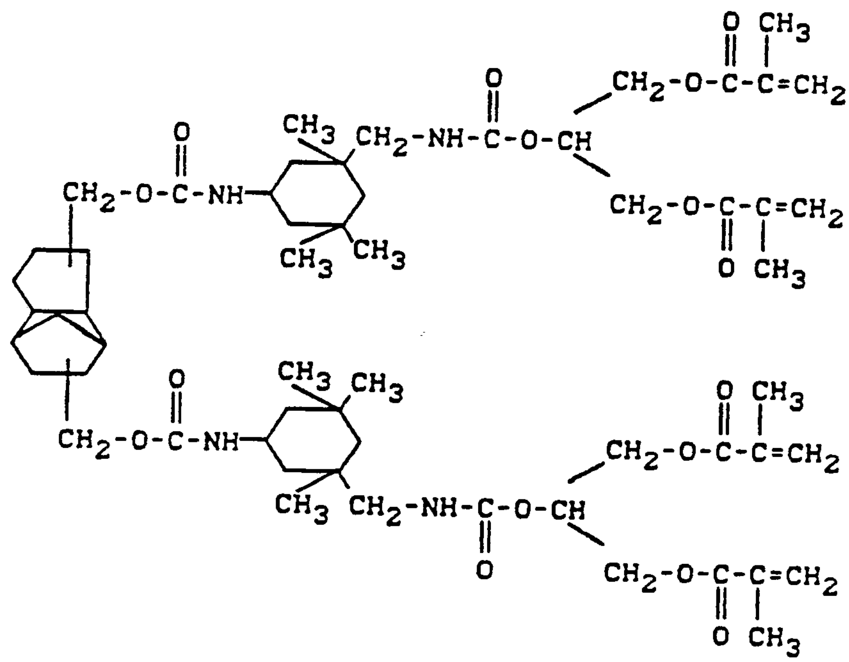

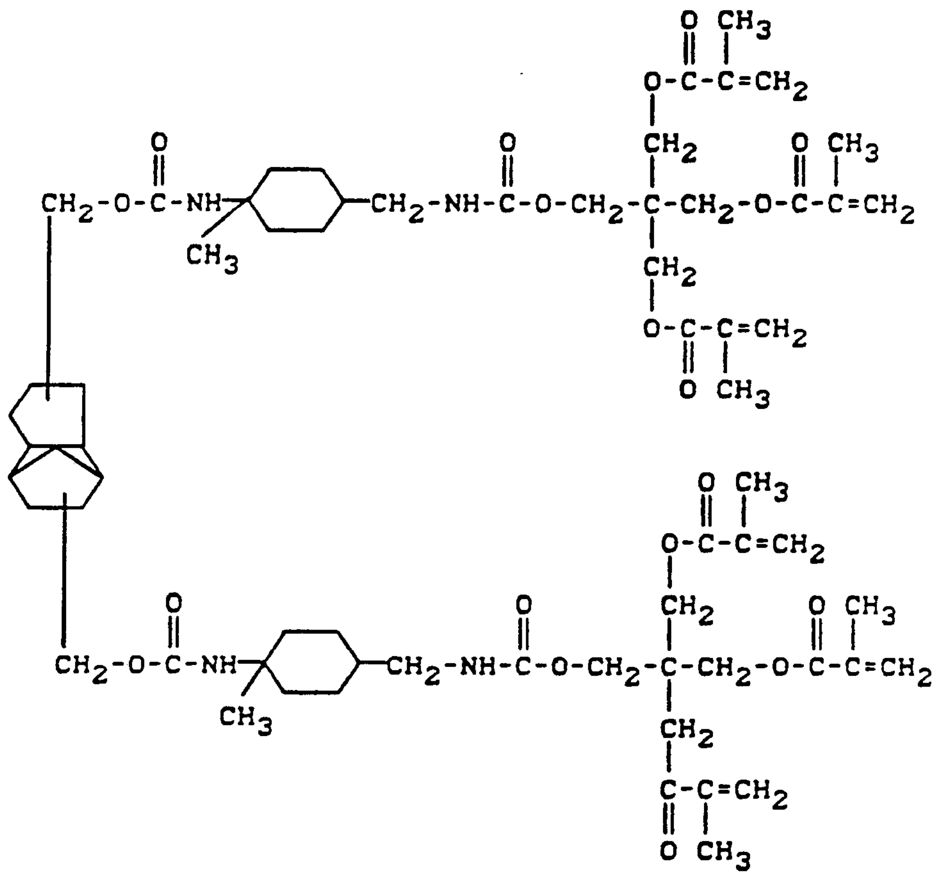

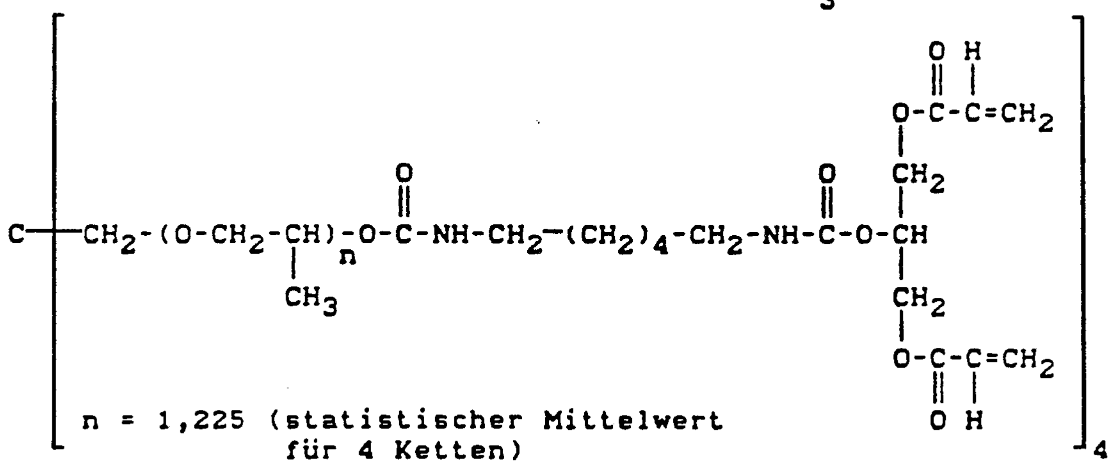

n = 1,225 (statistischer Mittelwert für 4 Ketten)

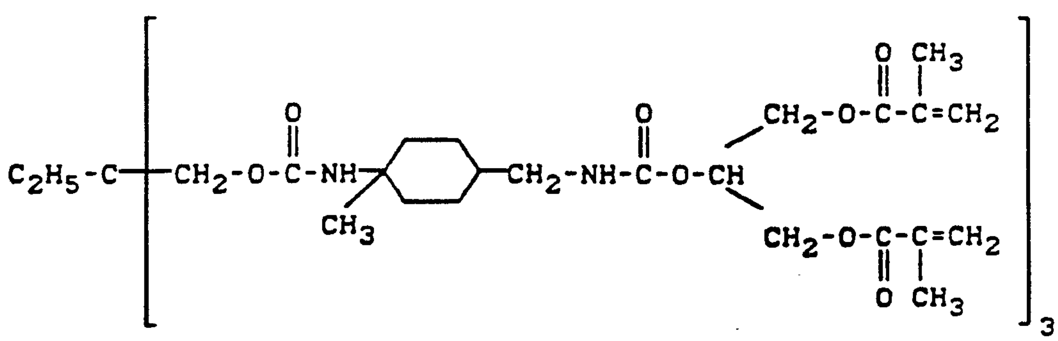

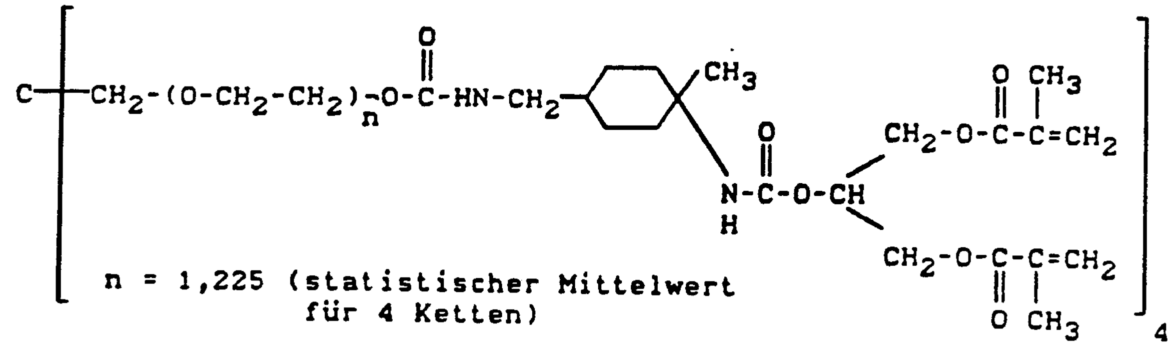

n = 1,225 (statistischer Mittelwert für 4 Ketten)

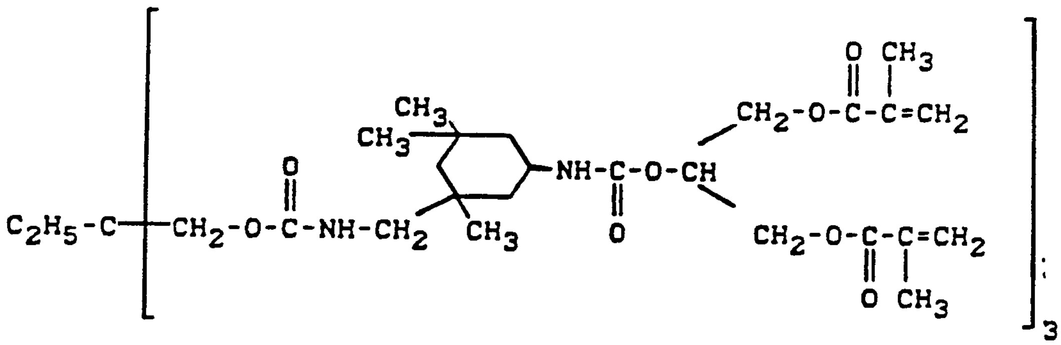

Es wurde auch ein Verfahren zur Herstellung der erfindungsgemäßen Urethangruppen enthaltenden (Meth)-acrylsäurederivate der Formel (I)

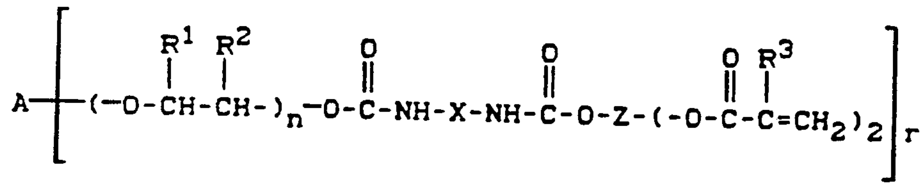
(I),

in der

A ein geradkettiger oder verzweigter, gegebenenfalls 1 bis 3 Sauerstoffbrücken enthaltender, aliphatischer Rest mit 2 bis 20 Kohlenstoffatomen, ein aromatischer Rest mit 6 bis 24 Kohlenstoffatomen, ein araliphatischer Rest mit 7 bis 26 Kohlenstoffatomen oder ein cycloaliphatischer Rest mit 6 bis 26 Kohlenstoffatomen ist,

r für die Anzahl der von A ausgehenden Ketten steht und eine Zahl von 2 bis 6 bedeutet,

$R^1$ und $R^2$ gleich sind und Wasserstoff oder verschieden sind und Wasserstoff und Methyl bedeuten,

n für jede von A ausgehende Kette unabhängig einer Zahl von 0 bis 5 bedeutet,

X ein zweiwertiger, geradkettiger oder verzweigter aliphatischer Rest mit 2 bis 24 Kohlenstoffatomen, ein aromatischer Rest mit 6 bis 26 Kohlenstoffatomen, ein araliphatischer Rest mit 7 bis 26 Kohlenstoffatomen oder ein monocycloaliphatischer Rest mit 6 bis 26 Kohlenstoffatomen bedeutet, wobei die aliphatischen, aromatischen, araliphatischen und/oder monocycloaliphatischen Reste 1 oder 2 Sauerstoffbrücken enthalten können, und wobei mehrere der aliphatischen, aromatischen, araliphatischen und/oder monocycloaliphatischen Reste über gegebenenfalls substituierte Methylengruppen verbunden sein können,

Z einen dreiwertigen geradkettigen oder verzweigten aliphatischen Kohlenwasserstoffrest mit 3 bis 15 Kohlenstoffatom, der gegebenenfalls 1 bis 3 Sauerstoffbrücken enthalten kann und gegebenenfalls durch 1 bis 3 (Meth)-acrylatreste substituiert sein kann, bedeutet, und

$R^3$ für jede von A ausgehende Kette unabhängig Wasserstoff oder Methyl bedeutet, dadurch gekennzeichnet, daß man einen Hydroxyalkyl-(Meth)-acrylsäureester der Formel (II)

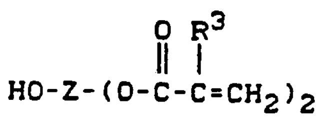
(II),

in der

Z und $R^3$ die obengenannte Bedeutung haben, mit einem Diisocyanat der Formel (III)

$$OCN—X—NCO \qquad (III),$$

in der

X die obengenannte Bedeutung hat, im Molverhältnis von etwa 1:1 bis 1:6 in einem inerten Lösungsmittel in Gegenwart eines katalysators umsetzt und dann das entstandene Isocyanatourethan nach Entfernen des nicht umgesetzten Diisocyanat mit einem Polyol der Formel (IV)

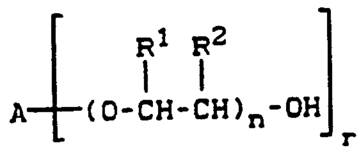

(IV),

in der

A, $R^1$, $R^2$, n und r die obengenannte Bedeutung haben, im Molverhältnis von OH-Gruppen zu NCO-Gruppen von etwa 1:1 umsetzt, gefunden.

(Meth)-acrylsäureester der Formel II sind an sich bekannt und können beispielsweise durch partielle Veresterung der entsprechenden Polyole erhalten werden.

Diisocyanate der Formel III sind an sich bekannt (EP 0 153 561) und können beispielsweise durch Umsetzung der Diamine mit Phosgen hergestellt werden.

Es kann zweckmäßig sein, das entstandene Isocyanaturethan zu reinigen, wenn das Diisocyant III im Überschuß bezogen auf Hydroxyalkyl(meth)acrylsäureester II eingesetzt wurde.

Die Reinigung des Isocyanaturethan erfolgt bevorzugt durch Extraktion mit aliphatischen Lösungsmitteln mit Siedepunkten unter 120°C bei Normaldruck, z.B. mit Pentan, n-Hexan, Isopentan.

Polyole der Formel IV sind an sich bekannt (Literatur z.B. DE—A 2 931 925) bzw. handelsüblich und können beispielsweise durch Oxyalkylierung der bekannten Polyole der Formel $A(OH)_r$, z.b. 2,2-Bishydroxymethylbutan, 2,2-Bishydroxymethyl-propan-1,3-diol, 3(4),8(9)-Bishydroxymethyl-tricyclo[5.2.1.$0^{2,6}$]decan usw. hergestellt werden. Durch die Herstellung bedingt, können die Polyole IV auch als Gemisch von Oxyalkylierungsprodukten mit variabler Kettenlänge vorliegen.

Für das erfindungsgemäße Verfahren werden im allgemeinen inerte Lösungsmittel verwendet. Beispielsweise seien Aceton, Chloroform, Tetrahydrofuran, Dioxan, Methylenchlorid, Toluol, Acetonitril genannt. Besonders bevorzugt sind Chloroform, Toluol, Acetonitril und Aceton.

Im allgemeinen wirde das erfindungsgemäße Verfahren unter Ausschluß von Wasser durchgeführt. Besonders bevorzugt wird eine maximale Menge an Wasser unter 0,1 Gew.-%, bezogen auf die Gesamtmenge der Reaktanden.

Katalysatoren für das erfindungsgemäße Verfahren sind im allgemeinen Metallsalze höherer Fettsäuren. Bevorzugte Katalysatoren können beispielsweise Dibutylzinnlaurat, Dibutylzinnmethoxid und Zipp-(II)-octoat sein. Als Katalysatoren können aber auch Verbindungen mit tertiären Aminogruppen, wie Triethylamin, Pyridin, 2-Methylpyridin, N,N-Dimethylpiperazin und N,N-Dimethyl-benzylamin verwendet werden. Es ist auch möglich, Titanverbindungen wie Tetrabutyl-titanat einzusetzen.

Im allgemeinen wird der Katalysator in einer Menge von 0,1 bis 2,5 Gew.-%, bevorzugt 0,1 bis 1,5 Gew.-%, bezogen auf die Gesamtmenge der Reaktanden eingesetzt werden.

In einer bevorzugten Ausführungsform kann das erfindungsgemäße Verfahren in Gegenwart eines Polymerisationsinhibitors durchgeführt werden. Polymerisationsinhibitoren sind an sich bekannt (Ullmanns Enzyklopädie der techn. Chemie, 4. Auflage, Verlag Chemie Weinheim, Band 8, Seiten 19—45). Beispielsweise seien 2,6-Di-tert.-butyl-4-methylphenol, Hydrochinon, Hydrochinonmonomethylether genannt.

Es ist auch möglich, als Polymerisationsinhibitor Sauerstoff, z.B. Luftsauerstoff, zu verwenden, der in das Raktionsgemisch eingeleitet wird.

Im allgemeinen wird der Polymerisationsinhibitor in einer Menge von 0,01 bis 1,0 Gew.-%, bevorzugt von 0,01 bis 0,2 Gew.-%, eingesetzt.

Die erste Stufe des erfindungsgemäßen Verfahrens wird im allgemeinen im Temperaturbereich von 0 bis 120°C, vorzugsweise von 30 bis 70°C, durchgeführt. Die zweite Stufe des erfindungsgemäßen Verfahrens wird im allgemeinen im Temperaturbereich von 0 bis 120°C, vorzugsweise von 30 bis 70°C, durchgeführt.

Das erfindungsgemäße Verfahren wird im allgemeinen bei Normaldruck durchgeführt. Es ist aber auch möglich, das erfindungsgemäße Verfahren bei Unter- oder Überdruck (beispielsweise im Druckbereich von 0,1 bis 10 bar) durchzuführen.

Das erfindungsgemäße Verfahren kann beispielsweise wie folgt durchgeführt werden:

Der (Meth)-acrylsäureester der Formel (II) und gegebenefalls der Polymerisationsinhibitor werden im inerten Lösungsmittel gelöst und unter Rühren zu dem gegebenenfalls gelösten Diisocyanat (III) zugetropft. Der Katalysator wird dabei einem der beiden Reaktanden zugesetzt. Die Reaktanden werden im Molverhältnis von etwa 1:1 bis 1:6 zur Umsetzung gebracht und bis zum vollständigen Umsatz der OH-Gruppen bzw. zum entsprechenden Umsatz der Isocyanatgruppen geführt. Die Umsetzung der Isocyanatgruppen kann in bekannter Weise durch IR-Spektroskopie und/oder durch Titration kontrolliert werden.

Ein Überschuß Diisocyanat kann anschließend mit n-Hexan, n-Pentan oder anderen aliphatischen Lösungsmitteln mit einem Siedepunkt unter 120°C (bei Normaldruck) extrahiert werden.

In der zweiten Stufe des erfindungsgemäßen Verfahrens wird das in der ersten stufe erhaltene Isocyanaturethan gegebenenfalls nach Extraktion eventuell vorhandenen überschüssigen Diisocyanats mit einem Polyol der Formel IV umgesetzt, so daß die Anzahl der Hydroxyläquivalente dels Polyols etwa der Anzahl der noch vorhandenen NCO-Äquivalente entspricht.

Bevorzugt werden $\frac{0,9}{r}$ bis $\frac{1,1}{r}$

Mol des Polyols IV, bezogen auf 1 Mol Hydroxyalkyl(meth)acrylat II, eingesetzt; r hat hierbei die oben angegebene Bedeutung einer Zahl von 2 bis 6.

Die Reaktion wird im allgemeinen bis zum vollständigen Umsatz geführt, so daß weder freies Isocyanat noch Polyol in der Umsetzung verbleiben. Nach beendeter Umsetzung wird das Reaktionsprodukt durch Entfernen des Lösungsmittels isoliert. Eine vorherige Filtration oder Reinigung mit Hilfe von Adsorbentien, bzw. Aktivkohle, Bleicherde, Kieselgel oder Aluminiumoxid ist möglich.

Nach dem erfindungsgemäßen Verfahren entsteht in der Regel ein Gemisch von Urethangruppen enthaltenden (Meth)-acrylsäurederivaten, die an Adsorbentien aufgetrennt werden können.

Für den erfindungsgemäßen Einsatz der neuen Urethan(meth)acrylate auf dem Dentalgebiet ist jedoch eine Auftrennung der erhaltenen Reaktionsgemische nicht erforderlich. Die gemische selbst können in vorteilhafter Weise als Komponente von Dentalwerkstoffen, beispielsweise Zahnfüllungsmaterialien, verwendet werden.

Mit Hilfe von Diisocyanaten mit unterschiedlicher Reaktivität der NCO-Gruppe ist es jedoch durchaus möglich, die erfindungsgemäßen Urethangruppen enthaltende (Meth)acrylsäurederivate der Formel I selektiv herzustellen. Hierfür geeignete Diisocyanate sind vor allem solche, die neben einer sterisch ungehinderten, aliphatisch gebundenen, eine sterisch gehinderte, cycloaliphatisch gebundene Isocyanatgruppen aufweisen, beispielsweise seien genannt:

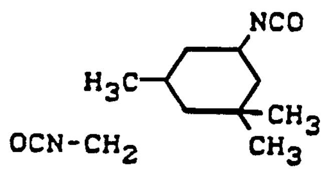

und

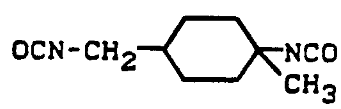

Bei Einsatz dieser Diisocyanate ergeben sich naturgemäß unterschiedliche Reaktionsgeschwindigkeiten für die erste und zweite Synthesestufe.

Es ist ebenfalls möglich, die erste und die zweite Stufe des oben genannten Verfahrens in ihrer Reihenfolge zu vertauschen. In diesem Fall werden in der ersten Stufe Diisocyanat III und polyol IV im Molverhältnis NCO:OH = 2 bis 10, vorzugsweise im Molverhältnis NCO:OH = 2,0 bis 4, bis zur Umsetzung aller Hydroxylgruppen in Urethangruppen zur Reaktion gebracht. Anschließend wird der vorhandene Überschuß an Diisocyanat (sofern dieses im Überschuß eingesetzt wurde) in der oben beschriebenen Weise mit den genannten Lösungsmitteln extrahiert. Die verbleibenden NCO-Gruppen, werden dann in der zweiten Stufe mit einem Hydroxyalkyl(meth)acrylat II zum erfindungsgemäßen (Meth)acrylsäureester umgesetzt. Bei Verwendung der Diisocyanate, deren NCO-Gruppen aufgrund sterischer Hinderung eine unterschiedliche Raktivität aufweisen, ist es vorteilhaft, eine Stöchiometrie von NCO:OH = 2,0—2,05 in der ersten Stufe einzuhalten.

Bei dieser Vedrfahrensvariante werden unter Verwendung des Diisocyanats der Formel

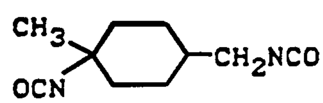

besonders einheitliche Produkte erhalten.

Die erfindungsgemäßen Urethan(meth)acrylsäurederivate können insbesondere als Monomere für Dentalwerkstoffe verwendet werden. Als Dentalwerkstoffe seien beispielsweise Füllungsmaterialien für Zähne, Beschichtungsmittel für Zähne und Komponenten für die Herstellung von Zahnersatz, bevorzugt Kunststoffzähne genannt. Je nach Anwendungsgebiet können Dentalwerkstoffe weitere Additive enthalten.

Für die Anwendung als Monomere für polymerisierbare Zahnfüllmassen oder Beschichtungsmittel im Dentalbereich können die erfindungsgemäßen (Meth)-acrylsäurederivate mit an sich bekannten Monomeren gemischt werden, um beispielsweise die Viskosität dem Verwendungszweck anzupassen. Hierbei werden Viskositäten im Bereich von 60 bis 10.000 mPas bevorzugt. Dieses ist dadurch erreichbar, daß man den erfindungsgemäßen Monomeren gegebenenfalls ein Comonomer niedriger Viskosität als Reaktivverdünner bzw. Lösungsmittel zumischt. Die erfindungsgemäßen Verbindungen sind in der Monomermischung mit einem Anteil von ca. 30 bis ca. 90 Gew.-%, bevorzugt von 40 bis 80 Gew.-%,

enthalten. Es ist auch bevorzugt, Mischungen verschiedener erfindungsgemäßer (Meth)-acrylsäurederivate einzusetzen.

Es ist auch möglich, Monomermischungen einzusetzen, die mehrere Comonomere enthalten, um die gewünschte Viskosität zu erreichen.

Beispielsweise seien die folgenden Comonomeren genannt: Glycerindi(meth)acrylat, Triethylenglykoldi(meth)acrylat, Tetraethylenglykoldi(meth)acrylat, 1,12-Dodecandioldi(meth)acrylat, 1,6-Hexandioldi(meth)acrylat, Diethylenglykol dimethacrylat, 2,2-Bis-[p-(2'-hydroxy-3'-methacryloyloxy propoxy)-phenyl]-propan, 2,2-Bis-[p-(2'-methacryloyloxyethoxy)-phenyl]-propan, Trimethylol-propan-tri-(meth)-acrylat, Bis-(meth)-acryloyloxyethoxymethyl-tricyclo-[5,2,1,$0^{2,6}$]-decan DE—A 29 31 925 und DE—A 29 31 926).

Insbesondere bevorzugt werden Comonomere, die bei 13 mbar einen Siedepunkt über 100°C besitzen.

Die erfindungsgemäßen polyfunktionellen (Meth)-acrylsäureester können gegebenenfalls in Mischung mit den genannten Comonomeren mit an sich bekannten Methoden zu vernetzten Polymerisaten aushärten (Am. Chem. Soc., Symp. Ser. *212*, 359—371 (1983)). Für die sogenannte Redoxpolymerisation ist ein System aus einer peroxidischen Verbindung und einem Reduktionsmittel, beispielsweise auf Basis tertiärer aromatischer Amine, geeignet. Beispiele für Peroxide sind: Dibenzoylperoxid, Dilauroylperoxid und Di-4-chlorbenzoylperoxid.

Als tertiäre aromatische Amine seien Beispielsweise N,N-Dimethyl-p-toluidin, Bis-(2-hydroxyethyl)-p-toluidin, Bis-(2-hydroxyethyl)-3,5-dimethylanilin und N-Methyl-N-(2-methylcarbamoyloxypropyl)-3,5-dimethylanilin genannt. Die Konzentrationen des Peroxids bzw. des Amins werden vorteil haft so gewählt, daß sie 0,1 bis 5 Gew.-%, bevorzugt 0,5 bis 3 Gew.-%, bezogen auf die Monomermischung betragen. Die peroxid- bzw. aminhaltigen Monomermischungen werden bis zur Anwendung getrennt gelagert.

Die erfindungsgemäßen Monomeren können auch durch Bestrahlung mit UV-Licht oder sichtbarem Licht (beispielsweise im Wellenlängenbereich von 230 bis 650 nm) zur Polymerisation gebracht werden. Als initiatoren für die photoinitiierte Polymerisation eignen sich beispielsweise Benzil, Benzildimethylketal, Benzoinmonoalkylether, Benzophenon, p-Methoxybenzophenon, Fluorenon, Thioxanthon, Phenanthrenchinon und 2,3-Bornandion (Campherchinon), gegebenenfalls in Gegenwart von synergistisch wirkenden Aktivatoren, wie N,N-Dimethylaminoethylmethacrylat, Triethanolamin, 4-N,N-Dimethylaminobenzolsulfonsäurediallylamid. Die Durchführung der Photopolymerisation ist beispielsweise in der DE—A 3 135 115 beschrieben.

Neben den oben beschriebenen Initiatoren können den erfindungsgemäßen (Meth)-acrylsäure-Derivaten an sich für diesen Einsatzzweck bekannte Lichtschutzmittel und Stabilisatoren zugesetzt werden.

Lichtschutzmittel sind beispielsweise in (Gächter, Müller, Taschenbuch der Kunststoff-Additive, 2. Ausgabe, Carl Hanser Verlag) beschrieben. Beispielsweise seien die folgenden lichtschutzmittel genannt: Cyasorb UV9®, Tinuvin P®, Tinuvin 770®, Tinuvin 622®, Tinuvin 765®.

Stabilisatoren sind beispielsweise in (Ullmanns Encyclopädie der technischen Chemie, 4. Auflage, Bd. 8) beschrieben. Beispielsweise seien die folgenden Stabilisatoren genannt; 2,6-Di-tert.-butylphenol, 2,6-Di-tert.-butyl-4-methylphenol, 2,6-Di-octadecyl-4-methyl-phenol, 1,1'-Methylen-bis(naphthol-2) u.a.

Die Lichtschutzmittel und die Stabilisatoren können jeweils in einer Menge von 0,01 bis 0,5 Gew.-Teilen, bezogen auf 100 Gew.-Teile der Monomermischung, eingesetzt werden.

Die Monomermischungen können ohne Zusatz von Füllstoffen als Beschichtungsmittel (Zahnlicke) eingesetzt werden.

Bei der Verwendung als Zahnfüllmassen setzt man den erhaltenen Monomermischungen im allgemeinen Füllstoffe zu. Um einen hohen Füllgrad erreichen zu können, sind Monomermischungen, die eine Viskosität im Bereich von 60 bis 10.000 mPas besitzen, besonders vorteilhaft.

Vorzugsweise mischt man den erfindungsgemäßen (Meth)acrylsäurederivaten anorganische Füllstoffe zu. Beispielsweise seien Bergkristall, Kristoballit, Quarzglas, hochdisperse Kieselsäure, Aluminiumoxid und Glaskeramiken, beispielsweise Lanthan und Zirkon enthaltende Glaskeramiken (DE—A—2 347 591) genannt. Die anorganischen Füllstoffe werden zur Verbesserung des Verbunds zur Polymermatrix des Polymethacrylats, vorzugsweise mit einem Haftvermittler vorbehandelt. Die Haftvermittlung kann beispielsweise durch eine Behandlung mit Organosiliciumverbindungen erreicht werden (Progress in Organic Coatings *11*, 297—308 (1983)). Bevorzugt wird 3-Methacryloyloxypropyl-trimethoxysilan eingesetzt. Die Füllstoffe für die erfindungsgemäßen Zahnfüllmassen weisen im allgemeinen einen mittleren Teilchendurchmesser von 0,01 bis 100 µm, vorzugsweise von 0,03 bis 50 µm, besonders bevorzugt von 0,03 bis 5 µm auf. Es kann auch vorteilhaft sein, mehrere Füllstoffe nebeneinander einzusetzen, die einen voneinander verschiedenen Teilchendurchmeser und/oder einen unterschiedlichen Silangehalt besitzen.

Der Füllstoffanteil in der Zahnfüllmasse beträgt im allgemeinen 5 bis 85 Gew.-%, vorzugsweise 50 bis 80 Gew.-%.

Für die Herstellung der Zahnfüllmassen werden die Komponenten unter Verwendung handelsüblicher Knetmaschinen vermischt.

Der Anteil der erfindungsgemäßen Urethan(meth)acrylate in den Zahnfüllmasen beträgt im allgemeinen 5 bis 70 Gew.-% bezogen auf die Füllmasse.

Die erfindungsgemäßen Zahnlacke und Zahnfüllmassen haben überraschenderweise einen besonders niedrigen Polymerisationsschrumpf und gute mechanische Festigkeiten, insbesondere eine hohe Härte

und hervorragende Verschleißfestigkeit.

Die erfindungsgemäßen Urethan(meth)acrylsäurederivate können auch als Komponenten bei der Herstellung von Zahnersatz eingesetzt werden.

Dabei werden die erfindungsgemäßen Monomeren mit den üblicherweise verwendeten, an sich bekannten Bestandteilen kombiniert. Vorzugsweise werden die Monomeren im Gemisch mit Alkylmethacrylaten, wie Methylmethacrylat eingesetzt. Es können auch zusätzlich an sich bekannte Perlpolymerisate zugesetzt werden. Zur einstellung der Zahnfarbe können bekannte anorganische und organische Farbpigmente und Trübungsmittel zugesetzt werden. Auch die Anwendung von Stabilisatoren und Lichtschutzmitteln ist möglich.

Die Kunststoffzähne werden durch radikalische Polymerisation der Dentalmassen unter Formgebung hergestellt.

Die Verarbeitung ist sowohl durch Injektionsverfahren als auch durch Prägeverfahren möglich und erfolgt im allgemeinen nach den Üblichen Herstellungsmethoden für Zähne auf Basis von Poly(methylmethacrylat), z.B. durch thermische Polymerisation unter Verwendung von an sich bekannten Polymerisationsinitiatoren, beispielsweise auf Basis von Peroxiden und Azoverbindungen, wie Dibenzoylperoxid, Dilauroylperoxid, Cyclohexylpercarbonat, Azobisisobuttersäurenitril. Gut geeignet sind auch Mischungen von Polymerisationsinitiatoren mit unterschiedlichen Zerfallstemperaturen.

Die aus den erfindungsgemäßen (Meth)acrylsäureestern hergestellten Dentalwerkstoffe zeichnen sich durch hohe Widerstandsfähigkeit gegenüber mechanischer Beanspruchung und eine hohe Abrasionsbeständigkeit aus.

Beispiel 1

Umsetzung von Glycerindimethacrylat mit Hexamethylendiisocyanat und Bis(hydroxymethyl)-tricyclo[5.2.1.0$^{2,6}$]decan (TCD—DM).

22,8 g handelsübliches Glycerindimethacrylat, 0,1 g Dibutylzinndilaurat und 20 mg 2,6-Di-tert.-butyl-4-methylphenol (Jonol) werden in 50 ml getrocknetem Toluol gelöst und zu 16,8 g Hexamethylendiisocyanat bei 50°C langsam zugetropft, wobei Luft durch den Reaktionsansatz geleitet wird. Anschließend wird bei 50°C so lange (ca. 3h) gerührt, bis die Hälfte der NCO-Gruppen umgesetzt ist. Die Bestimmung der NCO-Gruppen erfolgt in bekannter Weise durch Umsetzung mit überschüssigem Dibutylamin und Rücktitration mit Salzsäure. Dann werden 19,6 g TCD—DM, gelöst in 30 ml getrocknetem Chloroform, zugetropft und so lange gerührt (ca. 18h) bis IR-spektroskopisch kein Isocyanat mehr nachweisbar ist.

Das Reaktionsgemisch wird über Aktivkohle filtriert und durch Abdampfen vom Lösungsmittel befreit. Es wird eine hochviskose Flüssigkeit erhalten.

Beispiel 2

Umsetzung von Glycerindimethacrylat mit Isophorondiisocyanat und Bis(hydroxymethyl)-tricyclo-[5.2.1.0$^{2,6}$]decan.

Beispiel 1 wurde wiederholt, wobei jedoch anstelle von Hexamethylendiisocyanat 22,2 g Isophorondiisocyanat eingesetzt wurde und in der 2. Reaktionsstufe eine Reaktionszeit von 28 h eingehalten wurde. Man erhält einen weißen Feststoff.

Beispiel 3

Umsetzung von Glycerindimethacrylat mit 1,6-Hexamethylendiisocyanat und Pentaerythrit.

45,6 g handelsübliches Glycerindimethacrylat, 0,1 g Dibutylzinndilaurat und 34 mg Jonol werden in 50 ml getrocknetem Aceton gelöst und zu 33,6 g Hexamethylen-diisocyanat bei 45—50°C zugetropft. Es wird bei dieser Temperatur gerührt, bis die Hälfte der NCO-Gruppen abreagiert hat.

Dann werden weitere 6,8 g Pentaerythrit in 50 ml Aceton zugegeben. Die Mischung wird (etwa 48 h) bei 50°C gerührt, bis im IR-Spektrum kein Isocyanat mehr detektierbar ist. Das Reaktionsprodukt wird über Aktivkohle filtriert und vom Lösungsmittel befreit.

Molmasse (osmometrisch): 1620 (berechnet: 1720)

200 MHz-$^1$H-NMR-Spektrum in $CDCl_3$/TMS (ppm]:

6,12/5,6 (=$CH_2$, m, 16H)

5,2—5,3 >(CH—O—, m, 4H)

H
|
4,2—4,4 (—O—*CH₂*—C—*CH₂*—O—, m, 16H)
|
O—

4,08 [(—O—$CH_2$)$_4$—C, S, 8H)

H<br>
/<br>
3,05—3,2 (—$CH_2$—N t, 16H)<br>
\\<br>
CO—O—,

1,92 (—C—$CH_3$, m, 24 H)<br>
‖<br>
$CH_2$

1,75 (—NH—, 8H)<br>
1,4—1,55/1,2—1,4 (—$(CH_2)_4$—, m, 32 H

Beispiel 4

Umsetzung von Glycerindimethacrylat mit 1,6-Hexamethylendiisocyanat und propoxyliertem Pentaerythrit.

45,6 g Glycerindimethacrylat, 39 mg Jonol und 0,1 g Dibutylzinndilaurat werden in 50 g getrocknetem Aceton gelöst und zu 33,6 g Hexamethylendiisocyanat zugetropft und bei 45—50°C gerührt, bis die Hälfte der NCO-Gruppen reagiert hat (ca. 3h). Durch Titration mit Dibutylamin und HCl wurde ein NCO-Gehalt von 6,5 Gewichtsprozent ermittelt.

Dann werden 20,1 g eines Addukts aus 4,9 Mol Propylenoxid und 1 Mol Pentaerythrit gelöst in 50 ml Aceton, zugegeben. Man hält bis zum vollständigen Umsatz der NCO-Gruppen (IR-spektroskopische Kontrolle) eine Reaktionstemperatur von 45—50°C aufrecht.

Nach beendeter Reaktion wird über Kieselgel filtriert und das Lösungsmittel am Rotationsverdampfer abgezogen. Das Produkt ist eine hochviskose, farblose Flüssigkeit.

Beispiel 5

21,0 g eines Adduktes aus 1 Mol Pentaerythrit und 4,9 Mol Propylenoxid (OH-Zahl = 534) und 5 mg Jonol werden in 100 ml Toluol auf 100°C erwärmt. Bei dieser Temperatur werden unter Rühren 38,8 g 4-Isocyanatomethyl-1-isocyanato-1-methyl-cyclohexan zugetropft. Man rührt die Reaktionsmischung ca. 46 Stunden bei 100°C, bis die Umsetzung der primären Isocyanatgruppen vollständig ist. Nach Abkühlen auf 40—50°C werden 45,6 g handelsübliches Glycerindimethacrylat und 0,1 g Zinnoktoat zugegeben. Man läßt bis zum vollständigen Umsatz der tertiären Isocyanatogruppen bei 50°C rühren und antfernt das Lösungsmittel (nach Filtration über Aktivkohle) bei $10^{-2}$ mm Hg und Raumtemperatur.

Das Produkt ist eine farblose, hochviskose Flüssigkeit.

Beispiel 6

Anwendungsstechnische Prüfungen

Die Urethan(acrylate) aus den Beispielen 1—4 sowie zu Vergleichszwecken das Monomer $A_1$ aus Beispiel 1 der GB 2 074 590, wurden mit Triethylenglycoldimethacrylat (TEGDMA) verdünnt und mit 0,5% N,N-Dimethylaminobenzolsulfonsäurebisallylamid, 0,2% Campherchinon und 0,125% Benzildimethylketal aktiviert. Die %-Angaben beziehen sich auf die Summe von Urethan(meth)acrylat und Triethylenglycoldimethacrylat. Die aktivierten Mischungen wurden mit einer handelsüblichen Dentallampe (Translux, Fa, Kulzer) zu festen Probekörpern ausgehärtet.

An diesen Probekörpern wurde die Biegefestigkeit nach DIN 13922 bestimmt sowie eine Härteprüfung nach der Wallacemethode durchgeführt.

Die Wallacemethode dient zur Bestimmung der Eindruckhärte an Kunststoffen. Ein Vickers-Diamant wird unter einer Vorlast von 1 p auf die Oberfläche aufgebracht und anschließend 60 Sek. lang mit einer Hauptlast von üblicherweise 100 p beansprucht. Als Maß für den Eindringwiderstand wird die Eindringtiefe des Diamanten unter Hauptlast gemessen. Im Gegensatz zu den Vickers- oder Brinellhärtemessungen, bei denen die Prüfkraft auf die Dimensionen des bleibenden Eindrucks bezogen wird, erfaßt man mit der Wallacemethode die elastische und die bleibende Verformung des Kunststoffes.

Diese Methode ist zur Charakterisierung von Werkstoffen für Anwendungen auf dem Dentalgebiet

besser geeignet als Härteprüfungen, die nur die bleibende Deformation erfassen. Je kleiner die Eindringtiefe $H_W$ ist, um so härter ist das Material.

| Urethan(meth)-acrylat | TEGDMA-Gehalt % | Biegefestigkeit [N/mm²] | $H_W$ (µm] |
|---|---|---|---|
| Monomer $A_1$ nach GB 2074590 (Vergleichsbeispiel) | 21,9 | 74 | 17,5 |
| Beispiel 1 | 36,8 | 83 | 14,1 |
| Beispiel 2 | 45,7 | 87 | 13,7 |
| Beispiel 3 | 45 | 95 | 13,0 |

Beispiel 7

Herstellung von Kunststoffzähnen

60 Gew.-Teile einer Monomermischung, die aus 45 Gew.-% Triethylenglykoldimethacrylat und aus 55 Gew.-% des Urethan-Methacrylsäurederivates aus Beispiel 4 hergestellt wurde, werden mit 1 Gew.-Teil Dibenzoylperoxid und 40 Gew.-Teilen einer mit 5% 3-Methacryloyloxypropyltrimethoxysilan silanisierten hochdispersen Kieselsäure (BET-Oberfläche: 50 m²/g vermischt.

Das aktivierte Gemisch wird in eine Zahnform eingespritzt und bei 130°C in 6 Minuten ausgehärtet. Die erhaltenen Kunststoffzähne zeigen eine besonders hohe Abrasionsfestigkeit.

**Patentansprüche**

1. Urethangruppen enthaltende (Meth)-acrylsäurederivate der Formel

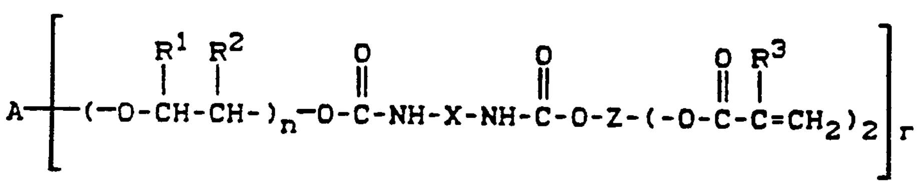

(I),

in der

A ein geradkettiger oder verzweigter, gegebenenfalls 1 bis 3 Sauerstoffbrücken enthaltender, aliphatischer Rest mit 2 bis 20 Kohlenstoffatomen, ein aromatischer Rest mit 6 bis 24 Kohlenstoffatomen, ein araliphatischer Rest mit 7 bis 26 Kohlenstoffatomen oder ein cycloaliphatischer Rest mit 6 bis 26 Kohlenstoffatomen ist,

r für die Anzahl der von A ausgehenden Ketten steht und eine Zahl von 2 bis 6 bedeutet,

$R^1$ und $R^2$ gleich sind und Wasserstoff oder verschieden sind und Wasserstoff und Methyl bedeuten,

n für jede von A ausgehende Kette unabhängig eine Zahl von 0 bis 5 bedeutet,

X ein zweiwertiger, geradkettiger oder verzweigter aliphatischer Rest mit 2 bis 24 Kohlenstoffatomen, ein aromatischer Rest mit 6 bis 26 Kohlenstoffatomen, ein araliphatischer Rest mit 7 bis 26 Kohlenstoffatomen oder ein monocycloaliphatischer Rest mit 6 bis 26 Kohlenstoffatomen ist, wobei die aliphatischen, aromatischen, araliphatischen und/oder monocycloaliphatischen Reste 1 oder 2 Sauerstoffbrücken enthalten können, und wobei mehrere der aliphatischen, aromatischen, araliphatischen und/oder monocycloaliphatischen Rest über gegebenenfalls substituierte Methylengruppen verbunden sein können,

Z einen dreiwertigen geradkettigen oder verzweigten aliphatischen Kohlenwasserstoffrest mit 3 bis 15 Kohlenstoffatomen, der gegebenenfalls 1 bis 3 Sauerstoffbrücken enthalten kann und gegebenenfalls durch 1 bis 3 (Meth)-acrylatreste substituiert sein kann, bedeutet, und

$R^3$ für jede von A ausgehende Kette unabhängig Wasserstoff oder Methyl beduetet.

2. Urethangruppen enthaltende (Meth)-acrylsäurederivate nach Anspruch 1, worin

A ein geradkettiger oder verzweigter, gegebenenfalls 1 bis 3 Sauerstoffbrücken enthaltender, aliphatischer Rest mit 3 bis 12 Kohlenstoffatomen, ein aromatischer Rest mit 6 bis 14 Kohlenstoffatomen, ein araliphatischer Rest mit 7 bis 26 Kohlenstoffatomen oder ein cycloaliphatischer Rest mit 6 bis 14 Kohlenstoffatomen ist,

r für die Anzahl der von A ausgehenden Ketten steht und eine Zahl von 2 bis 6 bedeutet,

$R^1$ und $R^2$ gleich sind und Wasserstoff oder verschieden sind und Wasserstoff und Methyl bedeuten,

n für jede von A ausgehende Kette unabhängig eine Zahl von 0 bis 5 bedeutet,

X ein zweiwertiger, geradkettiger oder verzweigter aliphatischer Rest mit 2 bis 12 Kohlenstoffatomen, oder ein monocycloaliphatischer Rest mit 6 bis 14 Kohlenstoffatomen, oder ein aromatischer Rest mit 6 bis 18 Kohlenstoffatomen, wobei auch 1 bis 3 der aliphatischen, monocycloaliphatischen oder aromatischen Reste über gegebenenfalls substituierte Methylengruppen verbunden sein können,

Z ein dreiwertiger geradkettiger oder verzweigter aliphatischer Kohlenwasserstoffrest mit 3 bis 10 Kohlenstoffatomen, der gegebenenfalls 1 oder 2 Sauerstoffbrücken enthalten kann und gegebenenfalls durch 1 oder 2 (Meth)-acrylatreste substituiert sein kann, ist, und

$R^3$ für jede von A ausgehende Kette unabhängig Wasserstoff oder Methyl beduetet.

3. Urethangruppen enthaltende (Meth)acrylsäurederivate nach den Ansprüchen 1 und 2, worin

A für den 2,2-Bismethylen-butan-1-yl-Rest, Propan-1,2,3-triyl-Rest, 2,2-Bismethylenpropan-1,3-diyl-Rest oder 3(4), 8(9)-Bismethylentricyclo[5.2.1.$0^{2,6}$]decan-Rest steht,

r für die Anzahl der von A ausgehenden Ketten steht und die Zahl 3 oder 4 bedeutet,

$R^1$ und $R^2$ gleich sind und Wasserstoff oder verschieden sind und Wasserstoff und Methyl bedeuten,

n für jede von A ausgehende Kette unabhängig eine Zahl von 0 bis 5 bedeutet,

X für einen der Reste

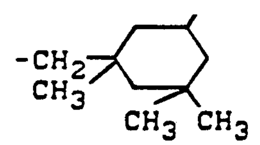

oder

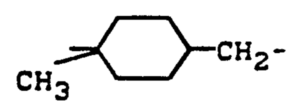

steht,

Z einen dreiwertigen geradkettigen oder verzweigten aliphatischen Kohlenwasserstoffrest mit 3 bis 10 Kohlenstoffatomen, der gegebenenfalls 1 Sauerstoffbrücke enthalten und gegebenenfalls durch 1 (Meth)acrylatrest substituiert sein kann, bedeutet,

$R^3$ für jede von A ausgehende Kette unabhängig Wasserstoff oder Methyl bedeutet.

4. Verfahren zur Herstellung von Urethangruppen enthaltende (Meth)-acrylsäurederivate der Formel

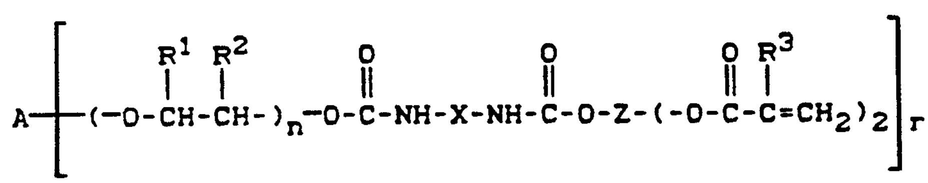

(I),

in der

A ein geradkettiger oder verzweigter, gegebenenfalls 1 bis 3 Sauerstoffbrücken enthaltender, aliphatischer Rest mit 2 bis 20 Kohlenstoffatomen, ein aromatischer Rest mit 6 bis 24 Kohlenstoffatomen, ein araliphatischer Rest mit 7 bis 26 Kohlenstoffatomen oder ein cycloaliphatischer Rest mit 6 bis 26 Kohlenstoffatomen ist,

r für die Anzahl der von A ausgehenden Ketten steht und eine Zahl von 2 bis 6 bedeutet,

$R^1$ und $R^2$ gleich sind und Wasserstoff oder verschieden sind und Wasserstoff und Methyl bedeuten,

n für jede von A ausgehende Kette unabhängig eine Zahl von 0 bis 5 bedeutet,

X ein zweiwertiger, geradkettiger oder verzweigter aliphatischer Rest mit 2 bis 24 Kohlenstoffatomen, ein aromatischer Rest mit 6 bis 26 Kohlenstoffatomen, ein araliphatischer Rest mit 7 bis 26 Kohlenstoffatomen oder ein monocycloaliphatischer Rest mit 6 bis 26 Kohlenstoffatomen, ist, wobei die aliphatischen, aromatischen, araliphatischen und/oder monocycloaliphatischen Reste 1 oder 2 Sauerstoffbrücken enthalten können, und wobei mehrere der aliphatischen, aromatischen, araliphatischen und/oder cycloaliphatischen Reste über gegebenenfalls substituierte Methylengruppen verbunden sein können,

Z einen dreiwertigen geradkettigen oder verzweigten aliphatischen Kohlenwasserstoffrest mit 3 bis 15 Kohlenstoffatomen, der gegebenenfalls 1 bis 3 Sauerstoffbrücken enthalten kann und gegebenenfalls durch 1 bis 3 (Meth)-acrylatreste substituiert sein kann, bedeutet, und

$R^3$ für jede von A ausgehende Kette unabhängig Wasserstoff oder Methyl bedeutet, dadurch gekennzeichnet, daß man einen (Meth)-acrylsäureester der Formel

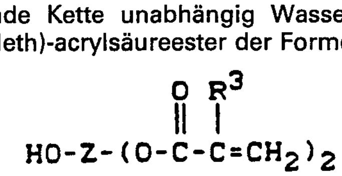

(II),

in der

Z und $R^3$ die obengenannte Bedeutung haben, mit einem Diisocyanat der Formel

OCN—X—NCO (III),

in der

X die obengenannte Bedeutung hat, im Molverhältnis von etwa 1:1 bis 1:6 in einem inerten Lösungsmittel in Gegenwart eines Katalysators umsetzt und dann das entstandene Isocyanatourethan nach Entfernen des nicht umgesetzten Diisocyanats mit einem Polyol der Formel (IV)

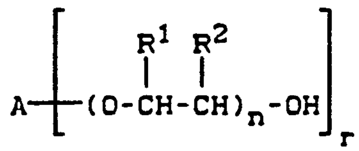

(IV),

in der

A, $R^1$, $R^2$, n und r die obengenannte Bedeutung haben, im Molverhältnis von OH-Gruppen zu NCO-Gruppen von etwa 1:1 umsetzt.

5. Verfahren nach Anspruch 4, dadurch gekennzeichnet, daß man die Umsetzung des (Meth)-acrylsäureesters mit dem Diisocyanat im Temperaturbereich von 0 bis 120°C durchführt.

6. Verfahren nach den Ansprüchen 4 und 5, dadurch gekennzeichnet, daß man die Umsetzung mit dem Polyol im Temperaturbereich von 0 bis 120°C durchführt.

7. Polymerisat aus Urethangruppen enthaltende (Meth)acrylsäurederivate der Formel

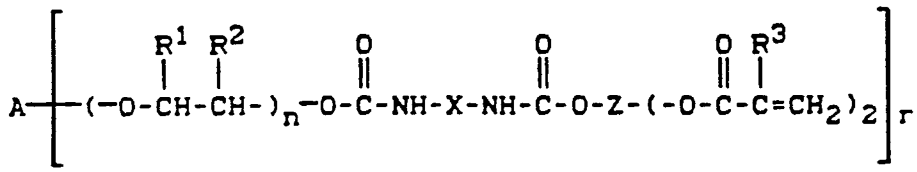

in der

A ein geradkettiger oder verzweigter, gegebenenfalls 1 bis 3 Sauerstoffbrücken enthaltender, aliphatischer Rest mit 2 bis 20 Kohlenstoffatomen, ein aromatischer Rest mit 6 bis 24 Kohlenstoffatomen, ein araliphatischer Rest mit 7 bis 26 Kohlenstoffatomen oder ein cycloaliphatischer Rest mit 6 bis 26 Kohlenstoffatomen ist,

r für die Anzahl der von A ausgehenden Ketten steht und eine Zahl von 2 bis 6 bedeutet,

$R^1$ und $R^2$ gleich sind und Wasserstoff oder verschieden sind und Wasserstoff und Methyl bedeuten,

n für jede von A ausgehende Kette unabhängig einer Zahl von 0 bis 5 bedeutet,

X ein zweiwertiger, geradkettiger oder verzweigter aliphatischer Rest mit 2 bis 24 Kohlenstoffatomen, ein aromatischer Rest mit 6 bis 26 Kohlenstoffatomen, ein araliphatischer Rest mit 7 bis 26 Kohlenstoffatomen oder ein monocycloaliphatischer Rest mit 6 bis 26 Kohlenstoffatomen ist, wobei die aliphatischen, aromatischen, araliphatischen und/oder monocycloaliphatischen Reste 1 oder 2 Sauerstoffbrücken enthalten können, und wobei mehrere der aliphatischen, aromatischen, araliphatischen und/oder monocycloaliphatischen Reste über gegebenenfalls substituierte Methylengruppen verbunden sein können,

Z einen dreiwertigen geradkettigen oder verzweigten aliphatischen Kohlenwasserstoffrest mit 3 bis 15 Kohlenstoffatom, der gegebenenfalls 1 bis 3 Sauerstoffbrücken enthalten kann und gegebenenfalls durch 1 bis 3 (Meth)-acrylatreste substituiert sein kann, bedeutet, und

$R^3$ für jede von A ausgehende Kette unabhängiges Wasserstoff oder Methyl bedeutet.

8. Verwendung von Urethangruppen enthaltende (Meth)-acrylsäurederivate der Formel

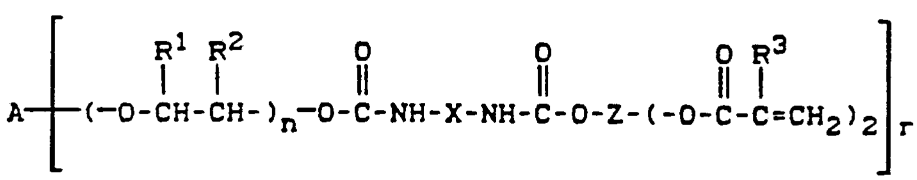

in der

A ein geradkettiger oder verzweigter, gegebenenfalls 1 bis 3 Sauerstoffbrücken enthaltender, aliphatischer Rest mit 2 bis 20 Kohlenstoffatomen, ein aromatischer Rest mit 6 bis 24 Kohlenstoffatomen, ein araliphatischer Rest mit 7 bis 26 Kohlenstoffatomen oder ein cycloaliphatischer Rest mit 6 bis 26 Kohlenstoffatomen ist,

r für die Anzahl der von A ausgehenden Ketten steht und eine Zahl von 2 bis 6 bedeutet,

$R^1$ und $R^2$ gleich sind und Wasserstoff oder verschieden sind und Wasserstoff und Methyl bedeuten,

n für jede von A ausgehende Kette unabhängig eine Zahl von 0 bis 5 bedeutet,

X ein zweiwertiger, geradkettiger oder verzweigter aliphatischer Rest mit 2 bis 24 Kohlenstoffatomen, ein aromatischer Rest mit 6 bis 26 Kohlenstoffatomen, ein araliphatischer Rest mit 7 bis 26 Kohlenstoffatomen oder ein monocycloaliphatischer Rest mit 6 bis 26 Kohlenstoffatomen ist, wobei die aliphatischen, aromatischen, araliphatischen und/oder monocycloaliphatischen Reste 1 oder 2 Sauerstoffbrücken enthalten können, und wobei mehrere der aliphatischen, aromatischen, araliphatischen und/oder monocycloaliphatischen Reste über gegebenenfalls substituierte Methylengruppen verbunden sein können,

Z einen dreiwertigen geradkettigen oder verzweigten aliphatischen Kohlenwasserstoffrest mit 3 bis 15 Kohlenstoffatomen, der gegebenenfalls 1 bis 3 Sauerstoffbrücken enthalten kann und gegebenenfalls durch 1 bis 3 (Meth)-acrylatreste substituiert sein kann, bedeutet, und

$R^3$ für jede von A ausgehende Kette unabhängig Wasserstoff oder Methyl bedeutet, in Dentalwerkstoffen.

9. Verwendung nach Anspruch 8, dadurch gekennzeichnet, daß die Urethangruppen enthaltenden (Meth)-acrylsäurederivate in Zahnfüllmassen eingesetzt werden.

10. Verwendung nach Anspruch 8, dadurch gekennzeichnet, daß die Urethangruppen enthaltenden (Meth)-acrylsäurederivate in Beschichtungsmittel für Zähne eingesetzt werden.

11. Verwendung nach Anspruch 8, dadurch gekennzeichnet, daß die Urethangruppen enthaltenden (Meth)-acrylsäurederivate für die Herstellung von Kunststoffzähnen eingesetzt werden.

12. Verwendung von Urethangruppen enthaltende (Meth)acrylsäurederivate der Formel

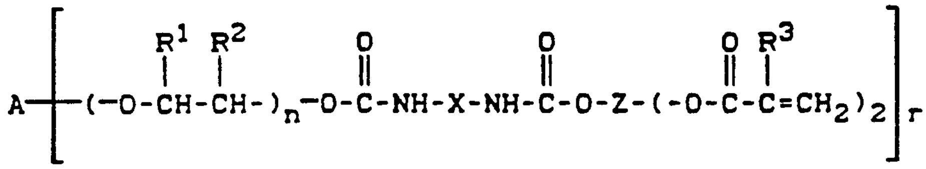

in der

A ein geradkettiger oder verzweigter, gegebenenfalls 1 bis 3 Sauerstoffbrücken enthaltender, aliphatischer Rest mit 2 bis 20 Kohlenstoffatomen, ein aromatischer Rest mit 6 bis 24 Kohlenstoffatomen, ein araliphatischer Rest mit 7 bis 26 Kohlenstoffatomen oder ein cycloaliphatischer Rest mit 6 bis 26 Kohlenstoffatomen ist,

r für die Anzahl der von A ausgehenden Ketten steht und eine Zahl von 2 bis 6 bedeutet,

$R^1$ und $R^2$ gleich sind und Wasserstoff oder verschieden sind und Wasserstoff und Methyl bedeuten,

n für jede von A ausgehende Kette unabhängig eine Zahl von 0 bis 5 bedeutet,

X ein zweiwertiger, geradkettiger oder verzweigter aliphatischer Rest mit 2 bis 24 Kohlenstoffatomen, ein aromatischer Rest mit 6 bis 26 Kohlenstoffatomen, ein araliphatischer Rest mit 7 bis 26 Kohlenstoffatomen oder ein monocycloaliphatischer Rest mit 6 bis 26 Kohlenstoffatomen ist, wobei die aliphatischen, aromatischen, araliphatischen und/oder monocycloaliphatischen Reste 1 oder 2 Sauerstoffbrücken enthalten können, und wobei mehrere der aliphatischen, aromatischen, araliphatischen und/oder monocycloaliphatischen Reste über gegebenenfalls substituierte Methylengruppen verbunden sein können,

Z einen dreiwertigen geradkettigen oder verzweigten aliphatischen Kohlenwasserstoffrest mit 3 bis 15 Kohlenstoffatomen, der gegebenenfalls 1 bis 3 Sauerstoffbrücken enthalten kann und gegebenenfalls durch 1 bis 3 (Meth)-acrylatreste substituiert sein kann, bedeutet, und

$R^3$ für jede von A ausgehende Kette unabhängig Wasserstoff oder Methyl bedeutet, zur Herstellung von Dentalwerkstoffen.

13. Dentalwerkstoffe, dadurch gekennzeichnet, daß sie Urethangruppen enthaltende (Meth)-acrylsäurederivate der Formel

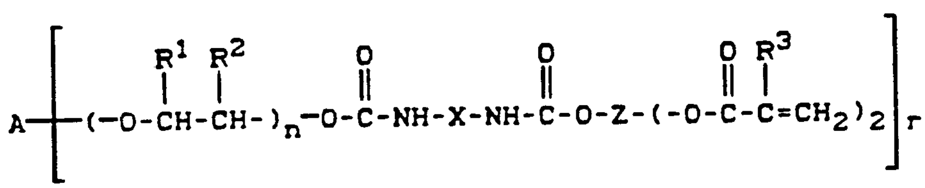

in der

A ein geradkettiger oder verzweigter, gegebenenfalls 1 bis 3 Sauerstoffbrücken enthaltender, aliphatischer Rest mit 2 bis 20 Kohlenstoffatomen, ein aromatischer Rest mit 6 bis 24 Kohlenstoffatomen, ein araliphatischer Rest mit 7 bis 26 Kohlenstoffatomen oder ein cycloaliphatischer Rest mit 6 bis 26 Kohlenstoffatomen ist,

r für die Anzahl der von A ausgehenden Ketten steht und eine Zahl von 2 bis 6 bedeutet,

$R^1$ und $R^2$ gleich sind und Wasserstoff oder verschieden sind und Wasserstoff und Methyl bedeuten,

n für jede von A ausgehende Kette unabhängig eine Zahl von 0 bis 5 bedeutet,

X ein zweiwertiger, geradkettiger oder verzweigter aliphatischer Rest mit 2 bis 24 Kohlenstoffatomen, ein aromatischer Rest mit 6 bis 26 Kohlenstoffatomen, ein araliphatischer Rest mit 7 bis 26 Kohlenstoffatomen oder ein monocycloaliphatischer Rest mit 6 bis 26 Kohlenstoffatomen ist, wobei die aliphatischen, aromatischen, araliphatischen und/oder monocycloaliphatischen Reste 1 oder 2 Sauerstoffbrücken enthalten können, und wobei mehrere der aliphatischen, aromatischen, araliphatischen und/oder monocycloaliphatischen Reste über gegebenenfalls substituierte Methylengruppen verbunden sein können,

Z einen dreiwertigen geradkettigen oder verzweigten aliphatischen Kohlenwasserstoffrest mit 3 bis 15 Kohlenstoffatomen, der gegebenenfalls 1 bis 3 Sauerstoffbrücken enthalten kann und gegebenenfalls durch 1 bis 3 (Meth)-acrylatreste substituiert sein kann, bedeutet, und

$R^3$ für jede von A ausgehende Kette unabhängig Wasserstoff oder Methyl bedeutet, enthalten.

14. Dentalwerkstoffe nach Anspruch 13, dadurch gekennzeichnet, daß sie neben Urethangruppen enthaltenden (Meth)-acrylsäurederivate weitere Comonomeren enthalten.

15. Dentalwerkstoffe nach den Ansprüchen 13 und 14, dadurch gekennzeichnet, daß sie neben Urethangruppen enthaltenden (Meth)-acrylsäurederivate und Comonomeren, an sich bekannte Additive und gegebenenfalls Füllstoffe enthalten.

**Revendications**

1. Dérivés d'acide (méth)-acrylique contenant des groupes uréthanne, de formule

$$A\left[(-O-\overset{R^1}{\overset{|}{C}H}-\overset{R^2}{\overset{|}{C}H}-)_n-O-\overset{O}{\overset{\|}{C}}-NH-X-NH-\overset{O}{\overset{\|}{C}}-O-Z-(-O-\overset{O}{\overset{\|}{C}}-\overset{R^3}{\overset{|}{C}}=CH_2)_2\right]_r \quad (I),$$

dans laquelle

A représente un radical aliphatique à chaîne droite ou ramifiée comprenant éventuellement 1 à 3 ponts d'oxygène, contenant de 2 à 20 atomes de carbone, un radical aromatique contenant de 6 à 24 atomes de carbone, un radical araliphatique contenant de 7 à 26 atomes de carbone ou un radical cycloaliphatique contenant de 6 à 26 atomes de carbone;

r représente le nombre de chaînes partant de A et désigne un chiffre de 2 à 6;

$R^1$ et $R^2$ sont identiques et représentent un atome d'hydrogène ou sont différents et représentent un atome d'hydrogène et un groupe méthyle;

n représente, pour chaque chaîne partant de A, de manière indépendante, un chiffre de 0 à 5;

X représente un radical aliphatique bivalent à chaîne droite ou ramifiée contenant 2 à 24 atomes de carbone, un radical aromatique contenant 6 à 26 atomes de carbone, un radical araliphatique contenant 7 à 26 atomes de carbone ou un radical monocycloaliphatique contenant 6 à 26 atomes de carbone, les radicaux aliphatiques, atomatiques, araliphatiques et/ou monocycloaliphatiques pouvant comprendre 1 ou 2 ponts d'oxygène et plusieurs des radicaux aliphatiques, aromatiques, araliphatiques et/ou monocycloaliphatiques pouvant être reliés par l'intermédiaire de groupes méthylène éventuellement substitués;

Z représente un radical d'hydrocarbure aliphatique trivalent à chaîne droite ou ramifiée, contenant 3 à 15 atomes de carbone, qui peut éventuellement comprendre 1 à 3 ponts d'oxygène et qui peut éventuellement être substitué par 1 à 3 radicaux (méth)-acrylate; et

$R^3$ représente, pour chaque chaîne partant de A, de manière indépendante, un atome d'hydrogène ou un groupe méthyle.

2. Dérivés d'acide (méth)-acrylique contenant des groupes uréthanne, selon la revendication 1, dans lesquels

A représente un radical aliphatique à chaîne droite ou ramifiée, comprenant éventuellement 1 à 3 ponts d'oxygène, contenant 3 à 12 atomes de carbone, un radical aromatique contenant de 6 à 24 atomes de carbone, un radical araliphatique contenant de 7 à 26 atomes de carbone ou un radical cycloaliphatique contenant de 6 à 14 atomes de carbone;

r représente le nombre de chaînes partant de A et désigne un chiffre de 2 à 6;

$R^1$ et $R^2$ sont identiques et représentent un atome d'hydrogène ou sont différents et représentent un atome d'hydrogène et un groupe méthyle;

n représente, pour chaque chaîne partant de A, de manière indépendante, un chiffre de 0 à 5;

X représente un radical aliphatique bivalent à chaîne droite ou ramifiée contenant 2 à 12 atomes de carbone ou bien un radical monocycloaliphatique contenant 6 à 14 atomes de carbone ou encore un radical aromatique contenant 6 à 18 atomes de carbone, 1 à 3 des radicaux aliphatiques, monocycloaliphatiques ou aromatiques pouvant également être liés par l'intermédiaire de groupes méthylène éventuellement substitués;

Z représente un radical d'hydrocarbure aliphatique trivalent à chaîne droite ou ramifiée, contenant 3 à 10 atomes de carbone, qui peut éventuellement comprendre 1 à 2 ponts d'oxygène et qui peut être éventuellement substitué par 1 à 2 radicaux (méth)-acrylate; et

$R^3$ représente, pour chaque chaîne partant de A, de manière indépendante, un atome d'hydrogène ou un groupe méthyle.

3. Dérivés d'acide (méth)-acrylique contenant des groupes uréthanne, selon les revendications 1 et 2, dans lesquels

A représente le radical 2,2-bisméthylène butan-1-yle, le radical propane-1,2,3-triyle, le radical 2,2-bisméthylène propane-1,3-diyle ou le radical 3(4),8(9)-bisméthylène tricyclo[5.2.1.$0^{2,6}$]décane;

r représente le nombre de chaînes partant de A et désigne le chiffre 3 ou 4;

$R^1$ et $R^2$ sont indentiques et représentent un atome d'hydrogène ou sont différents et représentent un atome d'hydrogène et un groupe méthyle;

n représente, pour chaque chaîne partant de A, de manière indépendante, un chiffre de 0 à 5;

X représente un des radicaux

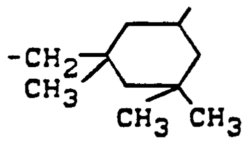

ou

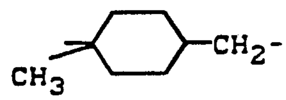

Z représente un radical d'hydrocarbure aliphatique trivalent à chaîne droite ou ramifiée, contenant 3 à 10 atomes de carbone, qui peut éventuellement contenir 1 pont oxygène et éventuellement être substitué par un radical (méth)-acrylate;

$R^3$ représente, pour chaque chaîne partant de A, de manière indépendante, un atome d'hydrogène ou un groupe méthyle.

4. Procédé pour la préparation de dérivés d'acide (méth)-acrylique contenant des groupes uréthanne, de formule

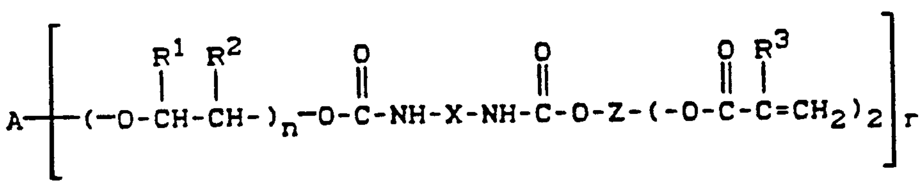

(I),

dans laquelle

A représente un radical aliphatique à chaîne droite ou ramifiée comprenant éventuellement 1 à 3 ponts d'oxygène, contenant de 2 à 20 atomes de carbone, un radical aromatique contenant de 6 à 24 atomes de carbone, un radical araliphatique contenant de 7 à 26 atomes de carbone ou un radical cycloaliphatique contenant de 6 à 26 atomes de carbone;

r représente le nombre de chaînes partant de A et désigne un chiffre de 2 à 6;

$R^1$ et $R^2$ sont identiques et représentent un atome d'hydrogène ou sont différents et représentent un atome d'hydrogène et un groupe méthyle;

n représente, pour chaque chaîne partant de A, de manière indépendante, un chiffre de 0 à 5;

X représente un radical aliphatique bivalent à chaîne droite ou ramifiée contenant 2 à 24 atomes de carbone, un radical aromatique contenant 6 à 26 atomes de carbone, un radical araliphatique contenant 7 à 26 atomes de carbone ou un radical monocycloaliphatique contenant 6 à 26 atomes de carbone, les radicaux aliphatiques, aromatiques, araliphatiques et/ou monocycloaliphatiques pouvant comprendre 1 ou 2 ponts d'oxygène et plusieurs des radicaux aliphatiques, aromatiques, araliphatiques et/ou monocycloaliphatiques pouvant être reliés par l'intermédiaire de groupes méthylène éventuellement substitués;

Z représente un radical d'hydrocarbure aliphatique trivalent à chaîne droite ou ramifiée contenant 3 à 15 atomes de carbone, qui peut éventuellement comprendre 1 à 3 ponts d'oxygène et qui peut éventuellement être substitué par 1 à 3 radicaux (méth)-acrylate; et

$R^3$ représente, pour chaque chaîne partant de A, de manière indépendante, un atome d'hydrogène ou un groupe méthyle; caractérisé en ce qu'on fait réagir un ester (méth)-acrylique de formule

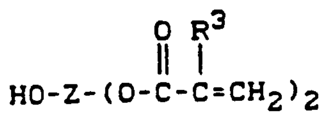

(II),

dans laquelle Z et $R^3$ ont la signification définie ci-dessus, avec un diisocyanate de formule

$$OCN—X—NCO \quad (III),$$

dans laquelle

X a la signification mentionnée ci dessus, dans un rapport molaire allant de 1:1 à 1:6, dans un solvant inerte, en présence d'un catalyseur, et ensuite on fait réagir l'isocyanatouréthanne obtenu, après élimination du diisocyanate n'ayant pas réagi, avec un polyol de formule (IV)

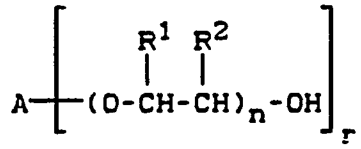

(IV),

dans laquelle A, $R^1$, $R^2$, n et r ont la signification définie ci-dessus, dans un rapport molaire des groupes OH aux groupes NCO d'environ 1:1.

5. Procédé selon la revendication 4, caractérisé en ce que l'on effectue la réaction de l'ester (méth)-acrylique avec le diisocyanate dans un domaine de températures allant de 0 à 120°C.

6. Procédé selon les revendications 4 et 5, caractérisé en ce que l'on effectue la réaction avec le polyol dans un domaine de températures allant de 0 à 120°C.

7. Polymère constitué de dérivés d'acide (méth)-acrylique contenant des groupes uréthanne, de formule

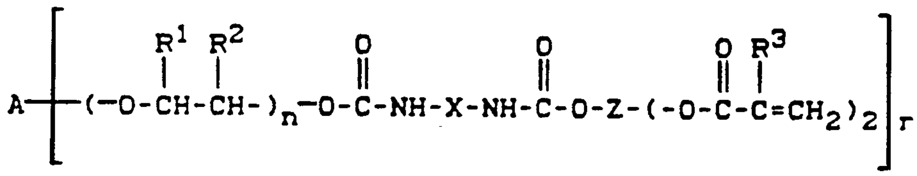

dans laquelle

A représente un radical aliphatique à chaîne droite ou ramifiée, comprenant éventuellement 1 à 3 ponts d'oxygène, contenant de 2 à 20 atomes de carbone, un radical aromatique contenant de 6 à 24 atomes de carbone, un radical araliphatique contenant de 7 à 26 atomes de carbone ou un radical cycloaliphatique contenant de 6 à 26 atomes de carbone;

r représente le nombre de chaînes partant de A et désigne un chiffre de 2 à 6;

$R^1$ et $R^2$ sont identiques et représentent un atome d'hydrogène ou sont différents et représentent un atome d'hydrogène et un groupe méthyle;

n représente, pour chaque chaîne partant de A, de manière indépendante, un chiffre de 0 à 5;

X représente un radical aliphatique bivalent à chaîne droite ou ramifiée contenant 2 à 24 atomes de carbone, un radical aromatique contenant 6 à 26 atomes de carbone, un radical araliphatique contenant 7 à 26 atomes de carbone ou un radical monocycloaliphatique contenant 6 à 26 atomes de carbone, les radicaux aliphatiques, aromatiques, araliphatiques et/ou monocycloaliphatiques pouvant comprendre 1 ou 2 ponts d'oxygène et plusieurs des radicaux aliphatiques, aromatiques, araliphatiques et/ou monocycloaliphatiques pouvant être reliés par l'intermédiaire de groupes méthylène éventuellement substitués;

Z représente un radical d'hydrocarbure aliphatique trivalent à chaîne droite ou ramifiée, contenant 3 à 15 atomes de carbone, qui peut éventuellement comprendre 1 à 3 ponts d'oxygène et qui peut éventuellement être substitué par 1 à 3 radicaux (méth)-acrylate; et

$R^3$ représente, pour chaque chaîne partant de A, de manière indépendante, un atome d'hydrogène ou un groupe méthyle.

8. Utilisation de dérivés d'acide (méth)acrylique contentant des groupes uréthanne, de formule

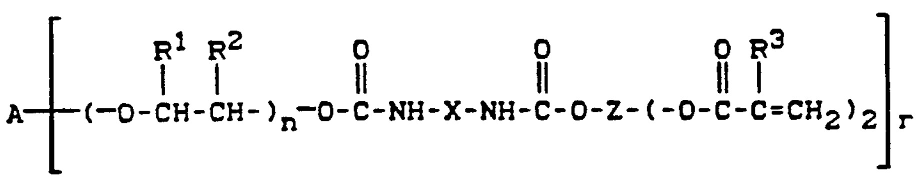

dans laquelle

A représente un radical aliphatique à chaîne droite ou ramifiée, comprenant éventuellement 1 à 3 ponts d'oxygène, contenant de 2 à 20 atomes de carbone, un radical aromatique contenant de 6 à 24 atomes de carbone, un radical araliphatique contenant de 7 à 26 atomes de carbone ou un radical cycloaliphatique contenant de 6 à 26 atomes de carbone;

r représente le nombre de chaînes partant de A et désigne un chiffre de 2 à 6;

$R^1$ et $R^2$ sont identiques et représentent un atome d'hydrogène ou sont différents et représentent un atome d'hydrogène et un groupe méthyle;

n représente, pour chaque chaîne partant de A, de manière indépendante, un chiffre de 0 à 5;

X représente un radical aliphatique bivalent à chaîne droite ou ramifiée contenant 2 à 24 atomes de

carbone, un radical aromatique contenant 6 à 26 atomes de carbone, un radical araliphatique contenant 7 à 26 atomes de carbone ou un radical monocycloaliphatique contenant 6 à 26 atomes de carbone, les radicaux aliphatiques, aromatiques, araliphatiques et/ou monocycloaliphatiques pouvant comprendre 1 ou 2 ponts d'oxygène et plusieurs des radicaux aliphatiques, aromatiques, araliphatiques et/ou monocycloaliphatiques pouvant être reliés par l'intermédiaire de groupes méthylène éventuellement substitués;

Z représente un radical d'hydrocarbure aliphatique trivalent à chaîne droite ou ramifiée, contenant 3 à 15 atomes de carbone, qui peut éventuellement comprendre 1 à 3 ponts d'oxygène et qui peut éventuellement être substitué par 1 à 3 radicaux (méth)-acrylate; et

$R^3$ représente, pour chaque chaîne partant de A, de manière indépendante, un atome d'hydrogène ou un groupe méthyle, dans des matières dentaires.

9. Utilisation selon la revendication 8, caractérisée en ce qu'on incorpore les dérivés (méth)-acryliques contenant des groupes uréthanne dans des matières de remplissage dentaire.

10. Utilisation selon la revendication 8, caractérisée en ce qu'on incorpore les dérivés (méth)-acryliques contenant des groupes uréthanne dans des agents d'enduction destinés à des dents.

11. Utilisation selon la revendication 8, caractérisée en ce que l'on met en oeuvre les dérivés (méth)-acryliques contenant des groupes uréthanne, pour la préparation de prothèses dentaires.

12. Utilisation de dérivés d'acide (méth)acrylique contenant des groupes uréthanne, de formule

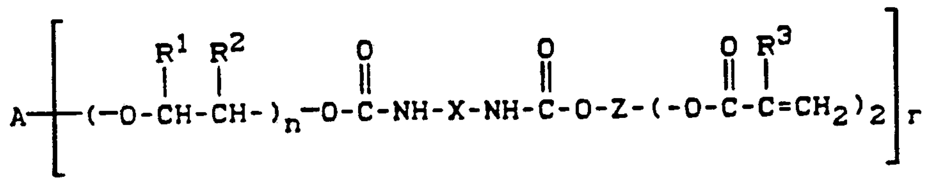

dans laquelle

A représente un radical aliphatique à chaîne droite ou ramifiée, comprenant éventuellement 1 à 3 ponts d'oxygène, contenant de 2 à 20 atomes de carbone, un radical aromatique contenant de 6 à 24 atomes de carbone, un radical araliphatique contenant de 7 à 26 atomes de carbone ou un radical cycloaliphatique contenant de 6 à 26 atomes de carbone;

r représente le nombre de chaînes partant de A et désigne un chiffre de 2 à 6;

$R^1$ et $R^2$ sont identiques et représentent un atome d'hydrogène ou sont différents et représentent un atome d'hydrogène et un groupe méthyle;

n représente, pour chaque chaîne partant de A, de manière indépendante, un chiffre de 0 à 5;

X représente un radical aliphatique bivalent à chaîne droite ou ramifiée contenant 2 à 24 atomes de carbone, un radical aromatique contenant 6 à 26 atomes de carbone, un radical araliphatique contenant 7 à 26 atomes de carbone ou un radical monocycloaliphatique contenant 6 à 26 atomes de carbone, les radicaux aliphatiques, aromatiques, araliphatiques et/ou monocycloaliphatiques pouvant comprendre 1 ou 2 ponts d'oxygène et plusieurs des radicaux aliphatiques, aromatiques, araliphatiques et/ou monocycloaliphatiques pouvant être reliés par l'intermédiaire de groupes méthylène éventuellement substitués;

Z représente un radical d'hydrocarbure aliphatique trivalent à chaîne droite ou ramifiée, contenant 3 à 15 atomes de carbone, qui peut éventuellement comprendre 1 à 3 ponts d'oxygène et qui peut éventuellement être substitué par 1 à 3 radicaux (méth)-acrylate; et

$R^3$ représente, pour chaque chaîne partant de A, de manière indépendante, un atome d'hydrogène ou un groupe méthyle, pour la préparation de matières dentaires.

13. Matières dentaires caractérisées en ce qu'elles contiennent des dérivés d'acide (méth)-acrylique contenant des groupes uréthanne, de formule

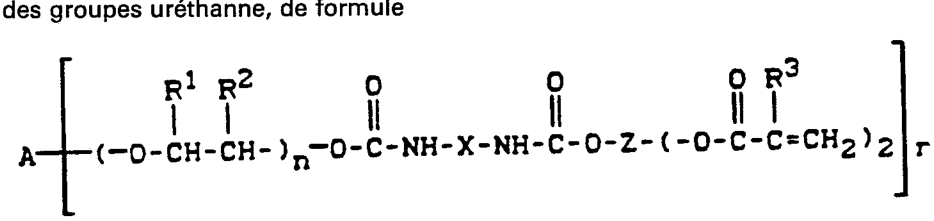

dans laquelle

A représente un radical aliphatique à chaîne droite ou ramifiée, comprenant éventuellement 1 à 3 ponts d'oxygène, contenant de 2 à 20 atomes de carbone, un radical aromatique contenant de 6 à 24 atomes de carbone, un radical araliphatique contenant de 7 à 26 atomes de carbone ou un radical cycloaliphatique contenant de 6 à 26 atomes de carbone;

r représente le nombre de chaînes partant de A et désigne un chiffre de 2 à 6;

$R^1$ et $R^2$ sont identiques et représentent un atome d'hydrogène ou sont différents et représentent un atome d'hydrogène et un groupe méthyle;

n représente, pour chaque chaîne partant de A, de manière indépendante, un chiffre de 0 à 5;

X représente un radical aliphatique bivalent à chaîne droite ou ramifiée contenant 2 à 24 atomes de carbone, un radical aromatique contenant 6 à 26 atomes de carbone, un radical araliphatique contenant 7 à

26 atomes de carbone ou un radical monocycloaliphatique contenant 6 à 26 atomes de carbone, les radicaux aliphatiques, aromatiques, araliphatiques et/ou monocycloaliphatiques pouvant comprendre 1 ou 2 ponts d'oxygène et plusieurs des radicaux aliphatiques, aromatiques, araliphatiques et/ou monocycloaliphatiques pouvant être reliés par l'intermédiaire de groupes méthylène éventuellement substitués;

Z représente un radical d'hydrocarbure aliphatique trivalent à chaîne droite ou ramifiée, contenant 3 à 15 atomes de carbone, qui peut éventuellement comprendre 1 à 3 ponts d'oxygène et qui peut éventuellement être substitué par 1 à 3 radicaux (méth)-acrylate; et

$R^3$ représente, pour chaque chaîne partant de A, de manière indépendante, un atome d'hydrogène ou un groupe méthyle.

14. Matières dentaires selon la revendication 13, caractérisée en ce que, outre les dérivés (méth)-acryliques contenant des groupes uréthanne, elles contiennent d'autres comonomères.

15. Matières dentaires selon les revendications 13 et 14, caractérisée en ce que, outre des dérivés (méth)-acryliques contenant des groupes uréthanne et des comonomères, elles contiennent des additifs connus et éventuellement des matières de remlissage.

**Claims**

1. (Meth)acrylic acid derivatives, containing urethane groups, of the formula

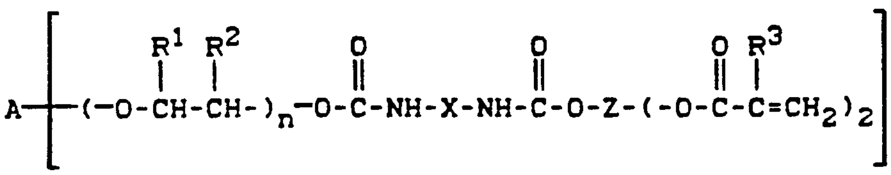

(I),

in which

A is a straight-chain or branched aliphatic radical having 2 to 20 carbon atoms and optionally containing 1 to 3 oxygen bridges, an aromatic radical having 6 to 24 carbon atoms, an araliphatic radical having 7 to 26 carbon atoms or a cycloaliphatic radical having 6 to 26 carbon atoms,

r represents the number of chains starting from A and denotes a number from 2 to 6,

$R^1$ and $R^2$ are identical and denote hydrogen or are different and denote hydrogen and methyl,

n denotes a number from 0 to 5, independently for each chain starting from A,

X is a divalent, straight-chain or branched aliphatic radical having 2 to 24 carbon atoms, an aromatic radical having 6 to 26 carbon atoms, an araliphatic radical having 7 to 26 carbon atoms or a monocycloaliphatic radical having 6 to 26 carbon atoms, it being possible for the aliphatic, aromatic, araliphatic and/or monocycloaliphatic radicals to contain 1 or 2 oxygen bridges and for several of the aliphatic, aromatic, araliphatic and/or monocycloaliphatic radicals to be linked via optionally substituted methylene groups,

Z denotes a trivalent, straight-chain or branched aliphatic hydrocarbon radical which has 3 to 15 carbon atoms, can optionally contain 1 to 3 oxygen bridges and can optionally be substituted by 1 to 3 (meth)acrylate radicals, and

$R^3$ denotes hydrogen or methyl, independently for each chain starting from A.

2. (Meth)acrylic acid derivatives containing urethane groups, according to Claim 1, wherein

A is a straight-chain or branched aliphatic radical having 3 to 12 carbon atoms and optionally containing 1 to 3 oxygen bridges, an aromatic radical having 6 to 14 carbon atoms, an araliphatic radical having 7 to 26 carbon atoms or a cycloaliphatic radical having 6 to 14 carbon atoms,

r represents the number of chains starting from A and denotes a number from 2 to 6,

$R^1$ and $R^2$ are identical and denote hydrogen or are different and denote hydrogen and methyl,

n denotes a number from 0 to 5, independently for each chain starting from A,

X is a divalent, straight-chain or branched aliphatic radical having 2 to 12 carbon atoms, a monocycloaliphatic radical having 6 to 14 carbon atoms or an aromatic radical having 6 to 18 carbon atoms, it also being possible for 1 to 3 of the aliphatic, monocycloaliphatic or aromatic radicals to be linked via optionally substituted methylene groups,

Z is a trivalent, straight-chain or branched aliphatic hydrocarbon radical having 3 to 10 carbon atoms, which can optionally contain 1 or 2 oxygen bridges and can optionally be substituted by 1 or 2 (meth)acrylate radicals, and

$R^3$ denotes hydrogen or methyl, independently for each chain starting from A.

3. (Meth)acrylic acid derivatives containing urethane groups, according to Claims 1 and 2, wherein

A represents the 2,2-bismethylene-butan-1-yl radical, propane-1,2,3-triyl radical, 2,2-bismethylenepropane-1,3-diyl radical or 3(4), 8(9)-bismethylenetricyclo[5.2.1.$0^{2.6}$]decane radical,

r represents the number of chains starting from A and denotes the numbere 3 or 4,

$R^1$ and $R^2$ are identical and denote hydrogen or are different and denote hydrogen and methyl,

n denotes a number from 0 to 5, independently for each chain starting from A,

X represents one of the radicals

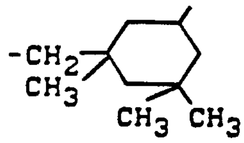

or

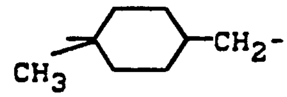

Z denotes a trivalent, straight-chain or branched aliphatic hydrocarbon radical which has 3 to 10 carbon atoms, can optionally contain 1 oxygen bridge and can optionally be substituted by 1 (meth)acrylate radical, and

$R^3$ denotes hydrogen or methyl, independently for each chain starting from A.

4. Process for the preparation of (meth)acrylic acid derivatives containing urethane groups, of the formula

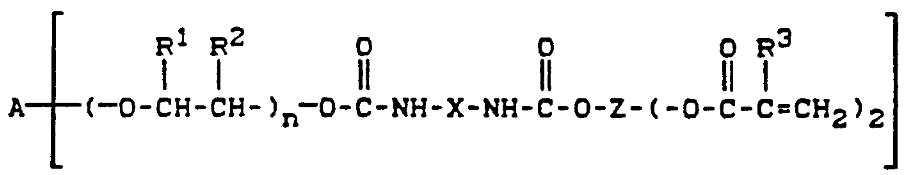 (I),

in which

A is a straight-chain or branched aliphatic radical having 2 to 20 carbon atoms and optionally containing 1 to 3 oxygen bridges, an aromatic radical having 6 to 24 carbon atoms, an araliphatic radical having 7 to 26 carbon atoms or a cycloaliphatic radical having 6 to 26 carbon atoms,

r represents the number of chains starting from A and denotes a number from 2 to 6,

$R^1$ and $R^2$ are identical and denote hydrogen or are different and denote hydrogen and methyl,

n denotes a number from 0 to 5, independently for each chain starting from A,

X is a divalent, straight-chain or branched aliphatic radical having 2 to 24 carbon atoms, an aromatic radical having 6 to 26 carbon atoms, an araliphatic radical having 7 to 26 carbon atoms or a monocycloaliphatic radical having 6 to 26 carbon atoms, it being possible for the aliphatic, aromatic, araliphatic and/or monocycloaliphatic radicals to contain 1 or 2 oxygen bridges and for several of the aliphatic, aromatic, araliphatic and/or cycloaliphatic radicals to be linked via optionally substituted methylene groups,

Z denotes a trivalent, straight-chain or branched aliphatic hydrocarbon radical which has 3 to 15 carbon atoms, can optionally contain 1 to 3 oxygen bridges and can optionally be substituted by 1 to 3 (meth)acrylate radicals, and

$R^3$ denotes hydrogen or methyl, independently for each chain starting from A, characterized in that a (meth)acrylic acid ester of the formula

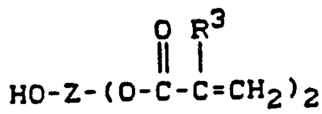 (II),

in which

Z and $R^3$ have the meaning given above, is reacted with a diisocyanate of the formula

$$OCN—X—NCO \quad (III),$$

in which

X has the meaning given above, in a molar ratio of about 1:1 to 1:6 in an inert solvent in the presence of a catalyst and the isocyanatourethane formed is then reacted, after the unconverted diisocyanate has been removed, with a polyol of the formula (IV)

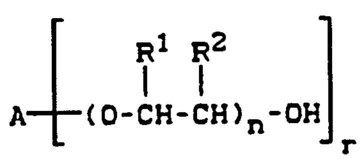 (IV),

in which

A, $R^1$, $R^2$, n and r have the meaning given above, is a molar ratio of OH groups to NCO groups of about 1:1.

5. Process according to Claim 4, characterized in that the reaction of the (meth)acrylic acid ester with the diisocyanate is carried out in the temperature range from 0 to 120°C.

6. Process according to Claims 4 and 5, characterized in that the reaction with the polyol is carried out in the temperature range from 0 to 120°C.

7. Polymer of (meth)acrylic acid derivatives containing urethane groups, of the formula

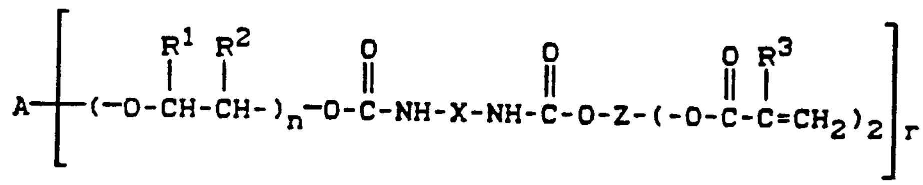

in which

A is a straight-chain or branched aliphatic radical having 2 to 20 carbon atoms and optionally containing 1 to 3 oxygen bridges, an aromatic radical having 6 to 24 carbon atoms, an araliphatic radical having 7 to 26 carbon atoms or a cycloaliphatic radical having 6 to 26 carbon atoms,

r represents the number of chains starting from A and denotes a number from 2 to 6,

$R^1$ and $R^2$ are identical and denote hydrogen or are different and denote hydrogen and methyl,

n denotes a number from 0 to 5, independently for each chain starting from A,

X is a divalent, straight-chain or branched aliphatic radical having 2 to 24 carbon atoms, an aromatic radical having 6 to 26 carbon atoms, an araliphatic radical having 7 to 26 carbon atoms or a monocycloaliphatic radical having 6 to 26 carbon atoms, it being possible for the aliphatic, aromatic, araliphatic and/or monocycloaliphatic radicals to contain 1 or 2 oxygen bridges and for several of the aliphatic, aromatic, araliphatic and/or monocycloaliphatic radicals to be linked via optionally substituted methylene groups,

Z denotes a trivalent, straight-chain or branched aliphatic hydrocarbon radical which has 3 to 15 carbon atoms, can optionally contain 1 to 3 oxygen bridges and can optionally be substituted by 1 to 3 (meth)acrylate radicals, and

$R^3$ denotes hydrogen or methyl, independently for each chain starting from A.

8. Use of (meth)acrylic acid derivatives containing urethane groups, of the formula

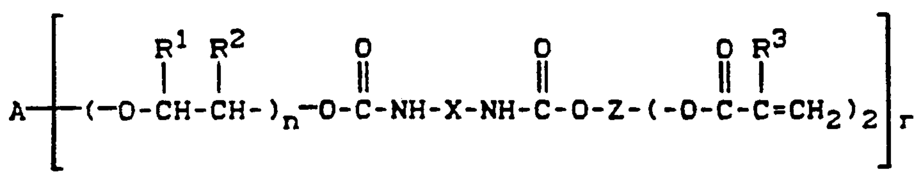

in which

A is a straight-chain or branched aliphatic radical having 2 to 20 carbon atoms and optionally containing 1 to 3 oxygen bridges, an aromatic radical having 6 to 24 carbon atoms, an araliphatic radical having 7 to 26 carbon atoms or a cycloaliphatic radical having 6 to 26 carbon atoms,

r represents the number of chains starting from A and denotes a number from 2 to 6,

$R^1$ and $R^2$ are identical and denote hydrogen or are different and denote hydrogen and methyl,

n denotes a number from 0 to 5, independently for each chain starting from A,

X is a divalent, straight-chain or branched aliphatic radical having 2 to 24 carbon atoms, an aromatic radical having 6 to 26 carbon atoms, an araliphatic radical having 7 to 26 carbon atoms or a monocycloaliphatic radical having 6 to 26 carbon atoms, it being possible for the aliphatic, aromatic, araliphatic and/or monocycloaliphatic radicals to contain 1 or 2 oxygen bridges and for several of the aliphatic, aromatic, araliphatic and/or monocycloaliphatic radicals to be linked via optionally substituted methylene groups,

Z denotes a trivalent, straight-chain or branched aliphatic hydrocarbon radical which has 3 to 15 carbon atoms, can optionally contain 1 to 3 oxygen bridges and can optionally be substituted by 1 to 3 (meth)acrylate radicals, and

$R^3$ denotes hydrogen or methyl, independently for each chain starting from A, in dental materials.

9. Use according to Claim 8, characterized in that the (meth)acrylic acid derivatives containing urethane groups are employed in tooth-filling compositions.

10. Use according to Claim 8, characterized in that the (meth)acrylic acid derivatives containing urethane groups are used in coating agents for teeth.

11. Use according to Claim 8, characterized in that the (meth)acrylic acid derivatives containing

urethane groups are employed for the preparation of plastic teeth.

12. Use of (meth)acrylic acid derivatives containing urethane groups, of the formula

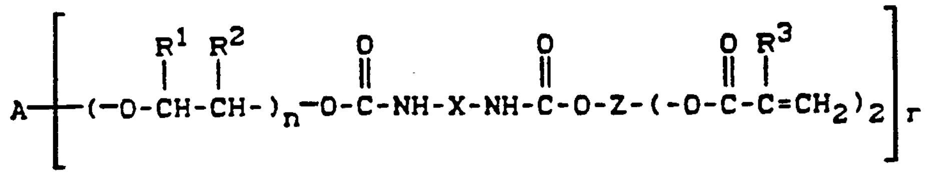

in which

A is a straight-chain or branched aliphatic radical having 2 to 20 carbon atoms and optionally containing 1 to 3 oxygen bridges, an aromatic radical having 6 to 24 carbon atoms, an araliphatic radical having 7 to 26 carbon atoms or a cycloaliphatic radical having 6 to 26 carbon atoms,

r represents the number of chains starting from A and denotes a number from 2 to 6,

$R^1$ and $R^2$ are identical and denote hydrogen or are different and denote hydrogen and methyl,

n denotes a number from 0 to 5, independently for each chain starting from A,

X is a divalent, straight-chain or branched aliphatic radical having 2 to 24 carbon atoms, an aromatic radical having 6 to 26 carbon atoms, an araliphatic radical having 7 to 26 carbon atoms or a monocycloaliphatic radical having 6 to 26 carbon atoms, it being possible for the aliphatic, aromatic, araliphatic and/or monocycloaliphatic radicals to contain 1 or 2 oxygen bridges and for several of the aliphatic, aromatic, araliphatic and/or monocycloaliphatic radicals to be linked via optionally substituted methylene groups,

Z denotes a trivalent, straight-chain or branched aliphatic hydrocarbon radical which has 3 to 15 carbon atoms, can optionally contain 1 to 3 oxygen bridges and can optionally be substituted by 1 to 3 (meth)acrylate radicals, and

$R^3$ denotes hydrogen or methyl, independently for each chain starting from A, for the preparation of dental materials.

13. Dental materials, characterized in that they contain (meth)acrylic acid derivatives containing urethane groups, of the formula

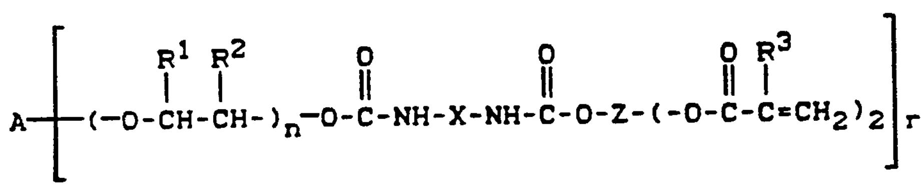

in which

A is a straight-chain or branched aliphatic radical having 2 to 20 carbon atoms and optionally containing 1 to 3 oxygen bridges, an aromatic radical having 6 to 24 carbon atoms, an araliphatic radical having 7 to 26 carbon atoms or a cycloaliphatic radical having 6 to 26 carbon atoms,

r represents the number of chains starting from A and denotes a number from 2 to 6,

$R^1$ and $R^2$ are identical and denote hydrogen or are different and denote hydrogen and methyl,

n denotes a number from 0 to 5, independently for each chain starting from A,

X is a divalent, straight-chain or branched aliphatic radical having 2 to 24 carbon atoms, an aromatic radical having 6 to 26 carbon atoms, an araliphatic radical having 7 to 26 carbon atoms or a monocycloaliphatic radical having 6 to 26 carbon atoms, it being possible for the aliphatic, aromatic, araliphatic and/or monocycloaliphatic radicals to contain 1 or 2 oxygen bridges and for several of the aliphatic, aromatic, araliphatic and/or monocycloaliphatic radicals to be linked via optionally substituted methylene groups,

Z denotes a trivalent, straight-chain or branched aliphatic hydrocarbon radical which has 3 to 15 carbon atoms, can optionally contain 1 to 3 oxygen bridges and can optionally be substituted by 1 to 3 (meth)acrylate radicals, and

$R^3$ denotes hydrogen or methyl, independently for each chain starting from A.

14. Dental materials according to Claim 13, characterized in that, in addition to (meth)acrylic acid derivatives containing urethane groups, they contain further comonomers.

15. Dental materials according to Claims 13 and 14, characterized in that, in addition to (meth)acrylic acid derivatives containing urethane groups, and comonomers, they contain additives known per se and, optionally, fillers.